(19) Europäisches Patentamt
European Patent Office
Office européen des brevets

(11) Publication number: **0 203 540**
**A1**

(12) # EUROPEAN PATENT APPLICATION

(21) Application number: **86106992.0**

(22) Date of filing: **23.05.86**

(51) Int. Cl.[4]: **C 07 C 103/50**, C 07 C 125/065, A 23 L 1/236

(30) Priority: **24.05.85 US 738112**

(43) Date of publication of application: **03.12.86 Bulletin 86/49**

(84) Designated Contracting States: **AT BE CH DE FR GB IT LI LU NL SE**

(71) Applicant: **GENERAL FOODS CORPORATION, 250 North Street, White Plains, N.Y. 10625 (US)**

(72) Inventor: **Roy, Glenn M., 16 Trimble Street, Garnerville, N.Y. (US)**
Inventor: **Barnett, Ronald E., 73 East Maple Avenue, Suffern, N.Y. (US)**
Inventor: **Zanno, Paul R., 2 April Lane, Nanuet, N.Y. (US)**

(74) Representative: **Füchsle, Klaus, Dipl.-Ing. et al, Hoffmann . Eitle & Partner Patentanwälte Arabellastrasse 4, D-8000 München 81 (DE)**

(54) **L-Aminodicarboxylic acid amides of substituted amines and a composition containing the same.**

(57) This invention relates to novel sweeteners of the formula:

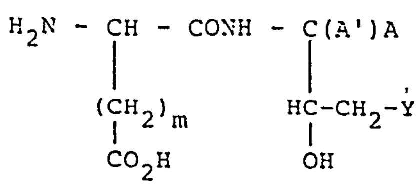

wherein

A is hydrogen, alkyl containing 1–3 carbon atoms, hydroxyalkyl containing 1–3 carbon atoms, alkoxymethyl wherein the alkoxy contains 1–3 carbon atoms or $CO_2R_5$ in which $R_5$ is alkyl containing 1–3 carbon atoms;

A' is hydrogen or alkyl containing 1–3 carbon atoms;

A and A' taken together with the carbon atoms to which they are attached form cycloalkyl containing 3–4 carbon atoms;

Y is $-(CHR)_n-R_1$, $-CHR_2R_3$ or $R_4$;

$R_1$ is cycloalkyl, cycloalkenyl, lower alkyl substituted cycloalkyl or cycloalkenyl, bicycloalkyl, bicycloalkenyl or tricycloalkyl containing up to 10 ring carbon atoms and up to a total of 12 carbon atoms;

R is H or alkyl containing 1–4 carbon atoms;

$R_2$ and $R_3$ are each cycloalkyl containing 3–4 ring carbon atoms;

$R_4$ is alkyl containing up to 12 carbon atoms;

n = 0 or 1; and

m = 0 or 1;

and food-acceptable salts thereof.

EP 0 203 540 A1

ACTORUM AG

# L-AMINODICARBOXYLIC ACID AMIDES OF SUBSTITUTED AMINES AND A COMPOSITION CONTAINING THE SAME

This invention relates to a novel group of compounds and more particularly to a novel group of compounds particularly well suited as sweeteners in edible foodstuff as well as a composition containing the same.

Sweetness is one of the primary taste cravings of both animals and humans. Thus, the utilization of sweetening agents in foods in order to satisfy this sensory desire is well established.

Naturally occuring carbohydrate sweeteners such as sucrose, are still the most widely used sweetening agents. While these naturally occurring carbohydrates, i.e., sugars, generally fulfill the requirements of sweet taste, the abundant usage thereof does not occur without deleterious consequence, e.g., high caloric intake and nutritional imbalance. In fact, often times the level of these sweeteners required in foodstuffs is far greater than the level of the sweetener that is desired for economic, dietetic or other functional consideration.

In an attempt to eliminate the disadvantages concomitant with natural sweeteners, considerable research and expense have been devoted to the production of artificial sweeteners, such as for example, saccharin, cyclamate, dihydrochalcone, aspartame, etc. While some of these artificial sweeteners satisfy the requirements of sweet taste without caloric input, and have met with considerable commercial success, they

are not, however, without their own inherent disadvantages. For example, many of these artificial sweeteners have the disadvantages of high cost, as well as delay in the perception of the sweet taste, persistent lingering of the sweet taste, and very objectionable bitter, metallic aftertaste when used in food products.

Since it is believed that many disadvantages of artificial sweeteners, particularly aftertaste, is a function of the concentration of the sweetener, it has been previously suggested that these effects could be reduced or eliminated by combining artificial sweeteners such as saccharin, with other ingredients such as aspartame or natural sugars, such as sorbitol, dextrose, maltose, etc. These combined products, however, have not been entirely satisfactory either. Some U.S. Patents which disclose sweetener mixtures include for example, U.S. Patent No. 4,228,198; U.S. Patent No. 4,158,068; U.S. Patent No. 4,154,862; and U.S. Patent No. 3,717,477.

Accordingly, much work has continued in an attempt to develop and identify compounds that have a sweet taste and which will satisfy the need for better lower calorie sweeteners. Search continues for sweeteners that have intense sweetness, that is, deliver a sweet taste at low use levels and which will also produce enough sweetness at low levels to act as sole sweetener for most sweetener applications. Furthermore, the sweeteners sought must have good temporal and sensory qualities. Sweeteners with good temporal qualities produce a time-intensity sweetness response similar to natural sweeteners without lingering. Sweeteners with good sensory qualities

lack undesirable off tastes and aftertaste. Furthermore, these compounds must be economical and safe to use.

In U.S. Patent No. 3,798,204, L-aspartyl-O-t-butyl-L-serine methyl ester and L-aspartyl-O-t-amyl-L-serine methyl ester are described as sweet compounds having significant sweetness.

In U.S. Patent No. 4,448,716 metal complex salts of dipeptide sweetners are disclosed. In the background of this patent a generic formula is described as an attempt to represent dipeptide sweeteners disclosed in five prior patents: U.S. Patent No. 3,475,403; U.S. Patent No. 3,492,131; Republic of South Africa Patent No. 695,083 published July 10, 1969; Republic of South Africa Patent No. 695,910 published August 14, 1969; and German Patent No. 2,054,554. The general formula attempting to represent these patents is as follows:

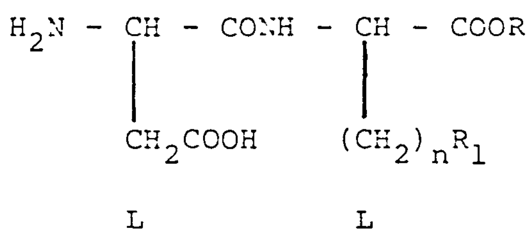

wherein R represents the lower alkyls, lower alkylaryls and cycloalkyls, n stands for integers 0 through 5, $R_1$ represents (a) phenyl group, (b) lower alkyls, (c) cycloalkyls, (d) $R_2$.

Where $R_2$ is hydroxy, lower alkoxy, lower alkyl, halogen, (e) $(S(O)_m$ (lower alkyl) where m is 0, 1 or 2 and provided n is 1 or 2, (f) $R_3$.

Where $R_3$ represents an hydroxy or alkoxy and (g) single or double unsaturated cycloalkyls with up to eight carbons. These compounds also are not entirely satisfactory in producing a high quality sweetness or in producing a sweet response at lower levels of sweetener.

Dipeptides of aspartyl-cysteine and aspartyl-methionine methyl esters are disclosed by Brussel, Peer and Van der Heijden in Chemical Senses and Flavour, 4, 141-152 (1979) and in Z. Lebensm. Untersuch-Forsch, 159, 337-343 (1975). The authors disclose the following dipeptides:

α-L-Asp-L-Cys(Me)-OMe
α-L-Asp-L-Cys(Et)-OMe
α-L-Asp-L-Cys(Pr)-OMe
α-L-Asp-L-Cys(i-Pr)-OMe
α-L-Asp-L-Cys(t-But)-OMe
α-L-Asp-L-Met-OMe

In U.S. Patent No. 4,399,163 to Brennan, et al. sweeteners having the following formulas are disclosed:

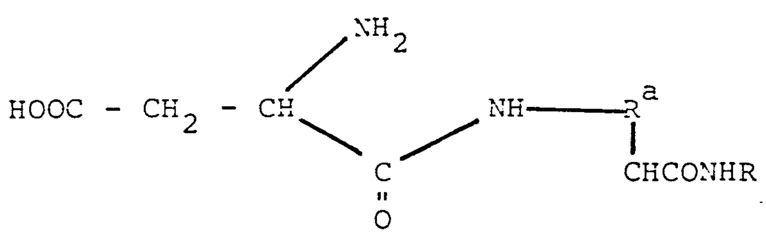

and physiologically acceptable cationic and acid addition salts thereof wherein

$R^a$ is $CH_2OH$ or $CH_2OCH_3$;

R is a branched member selected from the group consisting of fenchyl, diisopropylcarbinyl, d-methyl-t-butylcarbinyl, d-ethyl-t-butyl-carbinyl, 2-methylthio-2,4-dimethyl-pentan-3-yl, di-t-butyl-carbinyl,

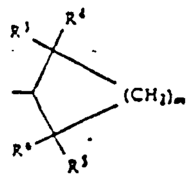

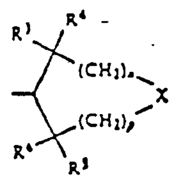

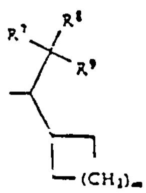

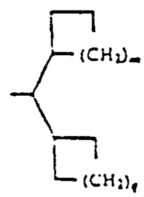

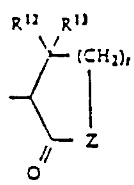

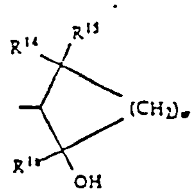

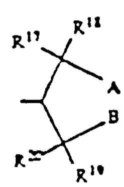

In a related patent, Patent No. 4,411,925, Brennan, et al. disclose compounds of the above general formula with R being defined hereinabove, except $R^a$ is defined as methyl, ethyl, n-propyl or isopropyl.

U.S. Patent No. 4,375,430 to Sklavounos discloses dipeptide sweeteners which are aromatic sulfonic acid salts of L-aspartyl-D-alaninoamides or L-aspartyl-D-serinamides.

European Patent Application No. 95772 to Tsau describe aspartyl dipeptide sweeteners of the formula:

$$H_2N - CHCONHCH - CO_2R'$$
$$HO_2C - CH_2 \quad R_2$$

wherein R' is alkyl of 1 to 6 carbons, and $R_2$ is phenyl, phenylalkenyl or cyclohexylalkenyl, wherein the alkenyl group has 1 to 5 carbons. Closely related is Patent No. 4,439,460 to Tsau, et al. which describes dipeptide sweeteners of the formula:

$$H_2N - CH - CONH - CH - COOR_1$$
$$CH_2 \qquad (CH_2)_n$$
$$CCOH \qquad R_2$$

wherein n is an integer from 0 to 5, and $R_1$ is an alkyl, alkylaryl or alicyclic radical. Similar such compounds are described in many related patents, the major difference being the definition of $R_2$.

In U.S. Patent No. 3,978,034 to Sheehan, et al. $R_2$ is defined as cycloalkenyl or phenyl. U.S. Patent No. 3,695,898 to Hill defines $R_2$ as a mono- or a di-unsaturated alicyclic radical. Haas, et al. in Patent No. 4,029,701 define $R_2$ as phenyl, lower alkyl or substituted or unsubstituted cycloalkyl, cycloalkenyl or cycloalkadienyl, or $S(O)_m$ lower alkyl provided that n is 1 or 2 and m is 0 or 2. Closely related are U.S. Patent Nos. 4,448,716; 4,153,737; 4,031,258; 3,962,468; 3,714,139; 3,642,491; and 3,795,746.

U.S. Patent No. 3,803,223 to Mazur, et al. describe dipeptide sweeteners and anti-inflammatory agents having the formula:

$$HOOC - CH_2\underset{\displaystyle NH_2}{CH} - \underset{\displaystyle O}{\overset{}{C}} - NRR'$$

wherein R is hydrogen or a methyl radical and R' is a radical selected from the group consisting of alkyl, or $\underset{\displaystyle X}{Alk} - Y$, wherein Alk is a lower alkylene radical, X is hydrogen or hydroxy, and Y is a radical selected from the group consisting of cyclohexyl, naphthyl, furyl, pyridyl, indolyl, phenyl and phenoxy.

Goldkamp, et al. in U.S. Patent No. 4,011,260 describe sweeteners of the formula:

$$\underset{\displaystyle COOH}{CH_2} - \underset{\displaystyle NH_2}{CH} - \overset{\displaystyle O}{C} - \overset{\displaystyle R}{N} - Alk - R'$$

wherein R is hydrogen or a lower alkyl radical, Alk is a lower alkylene radical and R' is a carbocyclic radical. Closely related is U.S. Patent No. 3,442,431.

U.S. Patent No. 4,423,029 to Rizzi describes sweeteners of the formula:

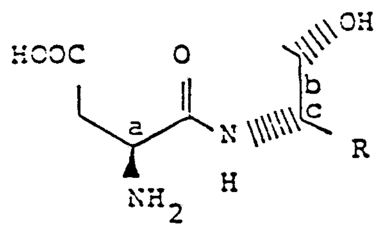

wherein R is $C_4$-$C_9$ straight, branched or cyclic alkyl, and wherein carbons a, b and c have the (S) configuration.

European Patent Application 48,051 describes dipeptide sweeteners of the formula:

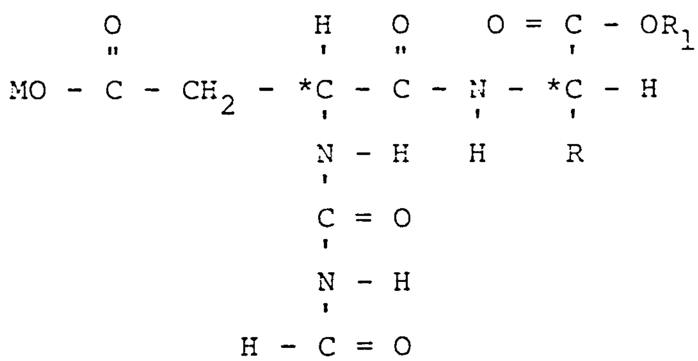

wherein M represents hydrogen, ammonium, alkali or alkaline earth,

R represents

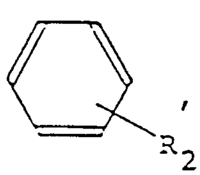 , 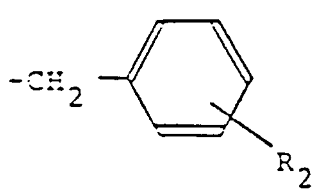 , 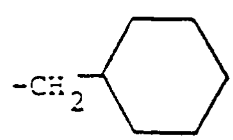

$R_1$ represents methyl, ethyl, propyl;

$R_2$ represents -OH, or $CH_3$;

* Signifies an L-optical configuration for this atom.

Dutch Patent Application No. 7207426 discloses L-aspartyl-3-fenchylalanine methyl ester as a sweetening agent.

U.S. Patent No. 3,971,822 to Chibata, et al., disclose sweeteners having the formula:

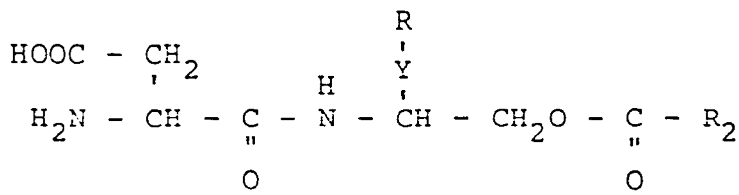

wherein R' is hydrogen or hydroxy, $R_2$ is alkyl of one to five carbon atoms, alkenyl of two to three carbon atoms, cycloalkyl of three to five carbon atoms or methyl cycloalkyl of four to six carbon atoms and Y is alkylene of one to four carbon atoms.

U.S. Patent No. 3,907,366 to Fujino, et al. discloses L-aspartyl-aminomalonic acid alkyl fenchyl diester and its' physiologically acceptable salts as useful sweeteners. Patent No. 3,959,245 disclose the 2-methyl cyclohexyl analog of the abovementioned patent.

U.S. Patent No. 3,920,626 discloses N-α L-aspartyl derivatives of lower alkyl esters of O-lower-alkanoyl-L-serine, β-alanine, γ-aminobutyric acid an D-β-aminobutyric acid as sweeteners.

Miyoshi, et al. in <u>Bulletin of Chemical Society of Japan</u>, <u>51</u>, p. 1433-1440 (1978) disclose compounds of the following formula as sweeteners:

```
HOOC - CH2             R'
       |               |
H2N -  CH - CONH -     CH - CH2 - COOR2
```

wherein R' is H, $CH_3$, $CO_2CH_4$, or benzyl and $R_2$ is lower alkyl or unsubstituted or substituted cycloalkyl.

European Patent Application 128,654 describes gem-diaminoalkane sweeteners of the formula:

```
                            R
                            |
H3N+ - CH - CONH -          C - NH - COR"
       |                    |
      (CH2)m                R'
       |
       CO2
```

wherein m is 0 or 1, R is lower alkyl (substituted or unsubstituted), R' is H or lower alkyl, and R" is a branched alkyl, alkylcycloalkyl, cycloalkyl, polycycloalkyl, phenyl, or alkyl-substituted phenyl, and physically acceptable salts thereof.

U.S. Patent No. 3,801,563 to Nakajima, et al. disclose sweeteners of the formula:

```
       COOH         COOR'
       |            |
       CH2         (CH2)n
       |            |
H2N -  CH - CONH -  CH - COOR2
```

wherein R' is a branched or cyclic alkyl group of 3 to 8 carbon atoms, $R_2$ is a lower alkyl group of 1 to 2 carbon atoms and n is a integer of 0 or 1.

BAD ORIGINAL

European Patent Application 34,876 describes amides of L-aspartyl-D-amino acid dipeptides of the formula:

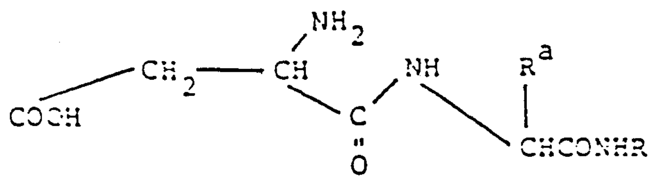

wherein $R^a$ is methyl, ethyl, n-propyl or isopropyl and R is a branched aliphatic, alicyclic or heterocyclic member which is branched at the alpha carbon atoms and also branched again at one or both of the beta carbon atoms. These compounds are indicated to be of significant sweetness.

In the Journal of Medicinal Chemistry, 1984, Vol. 27, No. 12, pp. 1663-8, are described various sweetener dipeptide esters, including L-aspartyl-α-aminocycloalkane methyl esters.

The various dipeptide esters of the prior art have been characterized as lacking significant stability at low pH values and/or thermal stability. These charac-terstics have limited the scope of use of these sweeteners in food products which are of low pH values or are prepared or served at elevated temperatures.

Accordingly, it is desired to find compounds that provide quality sweetness when added to foodstuffs or pharmaceuticals at low levels and thus eliminate or greatly diminish the aforesaid disadvantages associated with prior art sweeteners.

BAD ORIGINAL

SUMMARY OF THE INVENTION

The present new compounds are amides of certain α-aminodicarboxylic acids and substituted amino compounds. They are lower calorie sweeteners that possess a high order of sweetness with pleasing taste and higher stability at acid pH and elevated temperatures compared to known dipeptide sweeteners. This invention relates to a compound represented by the formula:

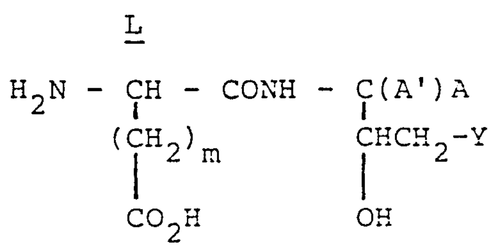

I

wherein

A is hydrogen, alkyl containing 1-3 carbon atoms, hydroxyalkyl containing 1-3 carbon atoms, alkoxymethyl wherein the alkoxy contains 1-3 carbon atoms or $CO_2R_5$ in which $R_5$ is alkyl containing 1-3 carbon atoms;

A' is hydrogen or alkyl containing 1-3 carbon atoms;

A and A' taken together with the carbon atom to which they are attached form cycloalkyl containing 3-4 carbon atoms;

Y is $-(CHR)_n-R_1$, $-CHR_2R_3$ or $R_4$;

$R_1$ is cycloalkyl, cycloalkenyl, lower alkyl substituted cycloalkyl or cycloalkenyl, bicycloalkyl, bicycloalkenyl or tricycloalkyl containing up to 10 ring carbon atoms and up to a total of 12 carbon atoms;

R is H or alkyl containing 1-4 carbon atoms;

$R_2$ and $R_3$ are each cycloalkyl containing 3-4 ring carbon atoms;

$R_4$ is alkyl containing up to 12 carbon atoms;

n = 0 or 1; and

m = 0 or 1;

and food-acceptable salts thereof.

This invention is also directed to new compounds of the formula:

```
B'HN - CH - CONH - C(A')A
       |           |
     (CH2)m      O=CCH2Y
       |
      CO2H
```

XI

wherein

A is hydrogen, alkyl containing 1-3 carbon atoms, hydroxyalkyl containing 1-3 carbon atoms, alkoxymethyl wherein the alkoxy contains 1-3 carbon atoms or $CO_2R_5$ in which $R_5$ is alkyl containing 1-3 carbon atoms;

A' is hydrogen or alkyl containing 1-3 carbon atoms

A and A' taken together with the carbon atoms to which they are attached form cycloalkyl containing 3-4 carbon atoms;

Y is $-(CHR)_n-R_1$, $-CHR_2R_3$ or $R_4$;

$R_1$ is cycloalkyl, cycloalkenyl, lower alkyl substituted cycloalkyl or cycloalkenyl, bicycloalkyl, bicycloalkenyl or tricycloalkyl containing up to 10 ring carbon atoms and up to a total of 12 carbon atoms;

R is H or alkyl containing 1-4 carbon atoms;

$R_2$ and $R_3$ are each cycloalkyl containing 3-4 ring carbon atoms;

$R_4$ is alkyl containing up to 12 carbon atoms;
B' is H or an amine protecting group;
n = 0 or 1; and
m = 0 or 1;
and food-acceptable salts thereof.

These compounds are useful intermediates in the preparation of compounds of Formula I.

DESCRIPTION OF THE PREFERRED EMBODIMENTS

In accordance with the present invention, the preferred compounds are those in which n = 0 and $R_1$ is an alkyl-substituted cycloalkyl or bicycloalkyl containing 5-7 ring carbon atoms and up to a total of 10 carbon atoms. Especially preferred are cycloalkyl substituted with at least one methyl group on the $\beta$ and/or $\beta'$ carbon atoms of the cycloalkyl ring. Particularly preferred cycloalkyls include cyclopropyl, cyclobutyl, cyclopentyl, and cyclohexyl and the preferred bicycloalkyl is fenchyl, patchanyl and nopanyl.

Also preferred are those compounds in which Y= $CHR_2R_3$, wherein $R_2$ and $R_3$ are each a cyclopropyl group; those in which Y is $R_4$ wherein $R_4$ is a branched alkyl such as di-t-butylmethyl, di-isopropylmethyl, and di-t-amylmethyl; and those in which Y is $-(CHR)_nR_1$ in which $R_1$ is cyclopropyl, R is a branched alkyl such as t-butyl and isopropyl and n=1.

The groups representative of $R_1$ in the present new compounds include such groups as cycloalkyl, e.g., cyclobutyl, cyclopentyl, cyclohexyl, cycloheptyl, etc.; alkyl-substituted cycloalkyls, e.g., 1-methylcyclopentyl, 1-methylcyclohexyl, 1-methylcyclobutyl, 1-methylcycloheptyl,

1,2-dimethylcycloheptyl, 2,3-dimethylcyclopentyl, 2,3-dimethylcyclohexyl, 2,3-dimethylcycloheptyl, 2,4-dimethylcyclopentyl, 2,4-dimethylcyclohexyl, 2,4-dimethylcycloheptyl, 2,5-dimethylcyclopentyl, 2,5-dimethylcyclohexyl, 2,5-dimethylcycloheptyl, 2,6-dimethylcyclohexyl, 2,6-dimethylcycloheptyl, 2,7-dimethylcycloheptyl, 3,5-dimethylcyclopentyl, 4,5-dimethylcyclopentyl, 4,5-dimethylcycloheptyl, 3,6-dimethylcyclohexyl, 3,7-dimethylcycloheptyl, 4,6-dimethylcyclohexyl, 4,7-dimethylcycloheptyl, 5,6-dimethylcyclohexyl, 5,6-dimethylcyclohexyl, 5,7-dimethylcycloheptyl, 6,7-dimethylcycloheptyl, 2,2-dimethylcyclopentyl, 2,2-dimethylcyclohexyl, 2,2-dimethylcycloheptyl, 2,2,3-trimethylcyclopentyl, 2,2,3-trimethylcyclohexyl, 2,2,3-trimethylcycloheptyl, 2,2,4-trimethylcyclopentyl, 2,2,4-trimethylcyclohexyl, 2,2,4-trimethylcycloheptyl, 2,2,5-trimethylcyclopentyl, 2,2,5-trimethylcyclohexyl, 2,2,5-trimethylcycloheptyl, 2,3,3-trimethylcyclopentyl, 2,3,3-trimethylcyclohexyl, 2,3,3-trimethylcycloheptyl, 2,4,4-trimethylcyclopentyl, 2,4,4-trimethylcyclohexyl, 2,4,4-trimethylcycloheptyl, 1,2,3-trimethylcyclopentyl, 1,2,3-trimethylcyclohexyl, 1,2,3-trimethylcycloheptyl, 1,2,4-trimethylcyclopentyl, 1,2,4-trimethylcyclohexyl, 1,2,4-trimethylcycloheptyl,

BAD ORIGINAL

1,2,5-trimethylcyclopentyl, 1,2,5-trimethylcyclohexyl, 1,2,5-trimethylcycloheptyl, 1,2,6-trimethylcyclohexyl, 1,2,6-trimethylcycloheptyl, 1,2,7-trimethylcycloheptyl, 2,3,4-trimethylcyclopentyl, 2,3,4-trimethylcyclohexyl, 2,3,4-trimethylcycloheptyl, 2,3,5-trimethylcyclopentyl, 2,3,5-trimethylcyclohexyl, 2,3,5-trimethylcycloheptyl, 2,3,6-trimethylcyclohexyl, 2,3,6-trimethylcycloheptyl, 2,3,7-trimethylcycloheptyl, 2,2,5,5-tetramethylcyclopentyl, 2,2,5,5-tetramethylcyclohexyl, 2,2,5,5-tetramethylcycloheptyl, 2,2,6,6-tetramethylcyclohexyl, 2,2,6,6-tetramethylcycloheptyl, 2,2,7,7-tetramethylcycloheptyl, 2,2,4,4-tetramethylcyclopentyl, 2,2,4,4-tetramethylcyclohexyl, 2,2,4,4-tetramethylcycloheptyl, 2,2,3,3-tetramethylcyclopentyl, 2,2,3,3-tetramethylcyclohexyl, 2,2,3,3-tetramethylcycloheptyl, 1,2,3,4-tetramethylcyclopentyl, 1,2,3,4-tetramethylcyclohexyl, 1,2,3,4-tetramethylcycloheptyl, 1,2,3,5-tetramethylcyclopentyl, 1,2,3,5-tetramethylcyclohexyl, 1,2,3,5-tetramethylcycloheptyl, 1,2,3,6-tetramethylcyclohexyl, 1,2,3,6-tetramethylcycloheptyl, 2,3,4,5-tetramethylcyclopentyl, 2,3,4,5-tetramethylcyclohexyl, 2,3,4,5-tetramethylcycloheptyl, 2,3,4,6-tetramethylcycloheptyl, 2,3,4,6-tetramethylcyclohexyl, 2,3,4,7-tetramethylcycloheptyl, 2,2,3,4-tetramethylcyclopentyl, 2,2,3,4-tetramethylcyclohexyl, 2,2,3,4-tetramethylcycloheptyl, 2,2,3,5-tetramethylcyclopentyl, 2,2,3,5-tetramethylcyclohexyl, 2,2,3,5-tetramethylcycloheptyl, 2,2,3,6-tetramethylcyclohexyl, 2,2,3,6-tetramethylcycloheptyl, 2,2,3,7-tetramethylcycloheptyl, 2,3,3,4-tetramethylcyclohexyl, 2,3,3,4-tetramethylcyclopentyl, 2,3,3,4-tetramethylcycloheptyl, 2,3,3,5-tetramethylcyclopentyl, 2,2,3,5-tetramethylcyclohexyl, 2,3,3,5-tetramethylcycloheptyl, 2,3,3,6-tetramethylcyclohexyl, 2,3,3,6-tetramethylcycloheptyl, 2,3,3,7-tetramethylcycloheptyl, 2,2,3,4-

BAD ORIGINAL

tetramethylcyclopentyl, 2,2,3,4-tetramethylcyclohexyl, 2,3,3,4-tetramethylcycloheptyl, 2,2,3,5-tetramethylcyclopentyl, 2,2,3,5-tetramethylcyclohexyl, 2,2,3,6-tetramethylcyclohexyl, 2,2,3,6-tetramethylcycloheptyl, 2,2,3,7-tetramethylcycloheptyl, 2,2,4,5-tetramethylcyclopentyl, 2,2,4,5-tetramethylcyclohexyl, 2,2,4,5-tetramethylcycloheptyl, 2,2,4,6-tetramethylcyclohexyl, 2,2,4,6-tetramethylcycloheptyl, 2,2,4,7-tetramethylcycloheptyl, dicyclopropylmethyl, t-butylcyclopropylmethyl, dicyclobutylmethyl, t-butylcyclobutylmethyl, etc.; $\beta$-alkyl-substituted cycloalkenes. e.g., 2-methyl-3-cyclohexenyl, 2-methyl-3-cyclopentenyl, 2-methyl-3-cycloheptenyl, 2-methyl-4-cycloheptenyl, 5-methyl-3-cyclopentenyl, 2-methyl-2-cyclopentenyl,. 2-methyl-2-cyclohexenyl, 2-methyl-2-cycloheptenyl, 2-methyl-2-cyclopentenyl, 6-methyl-2-cyclohexenyl, 7-methyl-2-cycloheptenyl, 2,3-dimethyl-2-cyclopentenyl, 2,3-dimethyl-2-cyclohexenyl, 2,4-dimethyl-2-cyclopentenyl, 2,4-dimethyl-2-cyclohexenyl, 2,5-dimethyl-2-cyclohexenyl, 2,5-dimethyl-2-cycloheptenyl, 2,6-dimethyl-2-cyclohexenyl, 2,6-dimethyl-3-cyclohexenyl, 2,5-dimethyl-3-cyclohexenyl, 2,5-dimethyl-2-cyclopentenyl, 2,4-dimethyl-3-cyclopentenyl, 2,4-dimethyl-3-cyclohexenyl, 4,5-dimethylcyclo-3-pentenyl, 5,5-dimethyl-3-cyclopentenyl, 6,6-dimethyl-3-cyclohexenyl, 1,2-dimethyl-3-cyclopentenyl, 1,2-dimethyl-3-cyclohexenyl, 1,5-dimethyl-3-cyclopentenyl, 2,2,6-trimethyl-3-cyclohexenyl, 2,2,5-trimethyl-3-cyclohexenyl, 2,5,5-trimethyl-3-cyclohexenyl, 2,7,7-trimethyl-3-cycloheptenyl, 2,7,7-trimethyl-4-cycloheptenyl, 2,2,7-trimethyl-3-cycloheptenyl, 2,2,7-trimethyl-4-cycloheptenyl, 2,3,6-trimethyl-3-cyclohexenyl, 2,3,7-trimethyl-3-cycloheptenyl, 2,3,5-trimethyl-3-cyclopentenyl, 2,2,6,6-tetramethyl-3-cyclohexenyl, 2,2,5,5-tetramethyl-3-cyclopentenyl, 2,2,7,7-tetramethyl-3-cycloheptenyl, 2,3,5,5-tetramethyl-3-cyclopentenyl, 2,3,6,6-tetramethyl-3-cyclohexenyl, 2,3,7,7-tetramethyl-3-cycloheptenyl, 2,3,6,6-tetramethyl-3-cycloheptenyl, 2,3,5,5-tetramethyl-3-cyclohexenyl, 2,3,4,5-tetramethyl-3-cyclopentenyl, 2,3,4,5-tetramethyl-3-

BAD ORIGINAL

cyclohexenyl, etc.; bicyclic compounds, such as norbornyl, norcaranyl, norpinanyl, bicyclo [2.2.2]-octyl, etc.; alkyl substituted bicyclic compounds, e.g., 6,6-dimethyl-bicyclo [3.1.1] heptyl, 6,7,7-trimethylnorbornyl (bornyl or camphanyl), pinanyl, thujanyl, caranyl, fenchyl, 2-norbornylmethyl, etc.; unsubstituted and alkyl-substituted bicyclo-alkenes such as norbornenyl, norpinenyl, norcarenyl, 2-(4-norbornenyl)methyl, pinenyl, carenyl, fenchenyl, etc.; and tricyclo compounds such as adamantyl and alkyl-substituted adamantyl, etc.

The preferred $R_1$ is cycloalkyl or bicycloalkyl or alkyl-substituted cycloalkyl or bicycloalkyl, especially where the alkyl group is in the $\beta$ or $\beta'$ positions. Further, preference exists for compound in which $R_1$ is a cycloalkyl with two, three or four alkyl groups in the $\beta$, $\beta'$ positions such as $\beta$, $\beta$, $\beta'$, $\beta'$-tetraalkyl-substituted cyclopentyl, cyclobutyl, cyclohexyl, and cycloheptyl, as well as $\beta$, $\beta$, $\beta'$-trialkyl substituted cyclobutyl, cyclopropyl, cyclohexyl, cyclopentyl, and cycloheptyl, and fenchyl. Also preferred are $\beta$-alkylcycloalkyls in which the alkyl group is isopropyl or tertiary butyl.

The alkyl groups representative of the substituent $R_4$ are normal or branched chain alkyl groups including, for example, ethyl, methyl, sec-butyl, t-butyl, t-amyl, sec-octyl, sec-butylmethyl, decyl, t-butylmethyl, sec-amyl, t-amyl, di-t-butylmethyl, diisopropylmethyl, di-t-amylmethyl, ethyl-t-butylmethyl, and similar alkyl groups. Particularly preferred alkyls are of the formula $-CH(R_6)(R_7)$ wherein $R_6$ is H or lower alkyl and $R_7$ is alkyl, preferably where $-CH(R_6)(R_7)$ contains at least 6 carbon atoms. Of these, the preferred are branched chain alkyl, particularly

BAD ORIGINA

tertiary alkyls. The most preferred are $-CH(R_6)(R_7)$ alkyls in which each of $R_6$ and $R_7$ is tertiary alkyl, e.g., t-butyl, or secondaryl alkyl, e.g., isopropyl.

In the groups $CO_2R_5$, $R_5$ is preferably methyl, but also includes ethvl, propyl and isopropyl.

The amine protecting group representative of the substituent B' in Formula XI is an electron-withdrawing protecting group. Exemplary protecting groups include $COCF_3$, $COCCl_3$, and CONAr-X, wherein Ar is aryl, X is $NO_2$, CN, COOR", COR", $SO_2R$", halo, carboxy, $SO_3H$, $SO_3R$", $SO_2NR$"R", $SO_2NH$ R", $SO_2NH_2$, CONR"R", CONHR", $CONH_2$, SOR", O-C(=O)-R", OR", $OSO_2R$", $OCF_3$, $CH_2OR$", $CH(OR")_2$, $COCF_3$, $CF_3$, $CH_2CF_3$, $CCl_3$, $C_tF_{2t+1}$, and the like;

wherein

each R" is the same or different and is $C_1$-$C_{12}$ alkyl and

t is an integer from 1-6.

Preferable X groups are CN, $COOC_2H_5$, $COOCH_3$, $SO_2CH_3$ or $COCH_3$ groups.

The aryl groups representative of Ar contain to 6-10 carbon and include such groups as phenyl, α-haphthyl or B-naphthyl,. Ar is preferably phenyl. It is preferable that X is located at the 4-position of the phenyl group.

These novel compounds are effective sweetness agents when used alone or in combination with other sweeteners in an ingesta, e.g., foodstuffs or pharmaceuticals. For example, other natural and/or artificial sweeteners which may be used with the novel compounds of the present invention include sucrose, fructose, corn syrup solids, dextrose, xylitol, sorbitol, mannitol, acetosulfam, thaumatin, invert sugar, saccharin, thiophene saccharin, meta-aminobenzoic acid, meta-hydroxybenzoic acid, cyclamate, chlorosucrose, dihydrochalcone, hydrogenated glucose syrups, aspartame (L-aspartyl-L-phenylalanine methyl ester) and other dipeptides, glycyrrhizin and stevioside and the like. These sweeteners when employed with the sweetness agents of the present invention, it is believed, could produce synergistic sweetness responses.

Furthermore, when the sweetness agents of the present invention are added to ingesta, the sweetness agents may be added alone or with nontoxic carriers such as the abovementioned sweeteners or other food ingredients such as acidulants and natural and artificial gums. Typical foodstuffs, and pharmaceutical preparations, in which the sweetness agents of the present invention may be used are, for example, beverages including soft drinks, carbonated beverages, ready to mix beverages and the like, infused foods (e.g. vegetables or fruits), sauces, condiments, salad dressings, juices, syrups, desserts, including puddings, gelatin and frozen

desserts, like ice creams, sherbets, icings and flavored frozen desserts on sticks, confections, toothpaste, mouthwash, chewing gum, cereals, baked goods, intermediate moisture foods (e.g. dog food) and the like.

In order to achieve the effects of the present invention, the compounds described herein are generally added to the food product at a level which is effective to perceive sweetness in the food stuff and suitably is in an amount in the range of from about 0.0005 to 2% by weight based on the consumed product. Greater amounts are operable but not practical. Preferred amounts are in the range of from about 0.001 to about 1% of the foodstuff. Generally, the sweetening effect provided by the present compounds are experienced over a wide pH range, e.g. 2 to 10 preferably 3 to 7 and in buffered and unbuffered formulations.

It is desired that when the sweetness agents of this invention are employed alone or in combination with another sweetner, the sweetner or combination of sweeteners provide a sucrose equivalent in the range of from about 2 weight percent to about 40 weight percent and more preferably from about 3 weight percent to about 15 weight percent in the foodstuff or pharmceutical.

A taste for determination of sweetness merely involves the determination of sucrose equivalency. Sucrose equivalence for sweeteners are readily determined. The amount of a sweetener that is equivalent to a given weight percent sucrose can be determined by having a panel of tasters taste solutions of a sweetener at known concentrations and match its sweetness to standard solutions of sucrose.

In order to prepare compounds of the present invention, several reaction schemes may be employed. In one reaction scheme, compounds of general formula II (a protected α-aminodicarboxylic acid or acylating derivatives thereof, e.g., acid halides, anhydrides, esters, etc.) and III are condensed to form compounds of general formula IV:

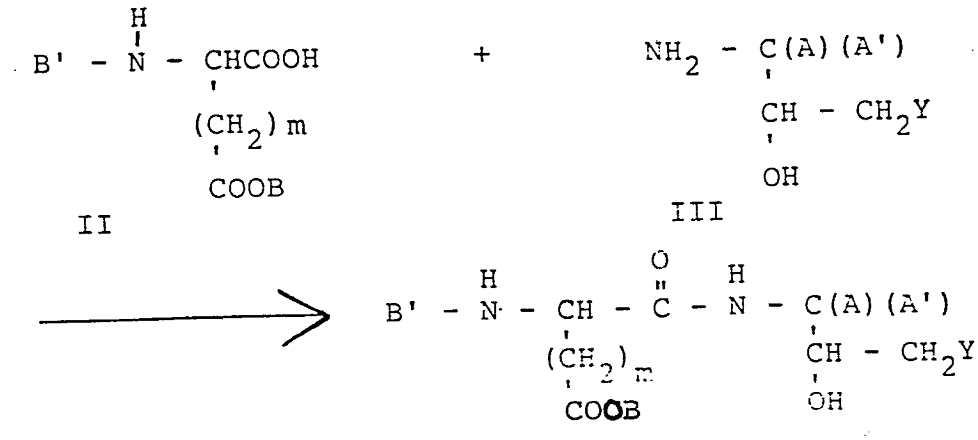

II III IV

In these, B' is an amino protecting group, B is a carboxy protecting group, and A, A', Y, and n have the same meaning as previously described. A variety of protecting groups known in the art may be employed. Examples of many of these possible groups may be found in "Protective Groups in Organic Synthesis" by T.W. Green, John Wiley and Sons, 1981. Among the preferred groups that may be employed are benzyloxycarbonyl for A and benzyl for B.

Coupling of compounds with general formula II to compounds having general formula III employs established techniques in peptide chemistry. One such technique uses dicyclohexylcarbodiimide (DCC) as the coupling agent. The DCC method may be employed with or without additives such as 4-dimethyl-aminopyridine or copper (II). The DCC coupling reaction generally proceeds at room temperature, however, it may be carried out from about -20° to 50°C. in a variety of solvents inert to the reactants. Thus suitable solvents include, but are not limited to, N,N-dimethyl-formamide, methylene chloride, toluene and the like. Preferably the reaction is carried out under an inert atmosphere such as argon or nitrogen. Coupling usually is complete within 2 hours but may take as long as 24 hours depending on reactants.

Various other amide-forming methods can be employed to prepare the desired compounds using suitable derivatives of the free-carboxy group in compounds of structure II, e.g., acid halide, mixed anhydride with acetic acid and similar derivatives. The following illustrates such methods using aspartic acid as the amino dicarboxylic acid.

One such method utilizes the reaction of N-protected aspartic anhydrides with the selected amino compound of formula III. Thus compounds of formula III can be reacted directly in inert organic solvents with L-aspartic anhydride having its amino group protected by a formyl, carbobenzloxy, or p-methoxycarbobenzloxy group which is subsequently removed after coupling to give compounds of general formula I. The N-acyl-L-aspartic anhydrides are prepared by reacting the corresponding acids with acetic anhydride in amounts of 1.0 - 1.2 moles per mole of the N-acyl-L-aspartic acid at 0° to 60°C in an inert solvent. The N-acyl-L-aspartic anhydrides are reacted with preferably 1 to 2 moles of compounds of formula III in an organic solvent capable of dissolving both and inert to the same. Representative solvents are ethyl acetate, methyl propionate, tetrahydrofuran, dioxane, ethyl ether, N,N-dimethylformamide and benzene. The reaction proceeds smoothly at 0° to 30°C. The N-acyl group is removed after coupling by catalytic hydrogenation with palladium on carbon or with HBr or HCl in a conventional manner. U.S. Patent 3,879,382 discloses that this coupling method can also be performed in an aqueous solvent at a temperature of -10° to 50°C. and at a pH of 4-12.

Alternatively, compounds of Formula I of the present invention can be prepared by reduction of the amido ketone of Formula V by using reducing agents known in the art, such as cerium borohydride followed by subsequent removal of the protecting groups, as follows:

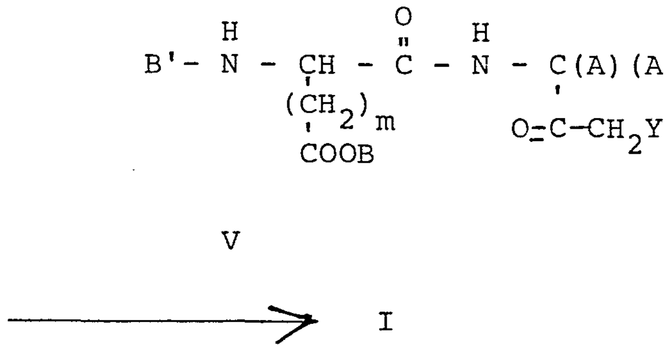

V

I

In these, B' is an amino protecting group, B is a carboxy protecting group, and A, A', Y, and n have the same meaning as previously described. A variety of protecting groups known in the art may be employed. Examples of many of these possible groups may be found in "Protective Groups in Organic Synthesis" by T.W. Green, John Wiley and Sons, 1981. Among the preferred groups that may be employed are benzyloxycarbonyl for A and benzyl for B.

Compound V can be prepared through a chiral synthetic pathway wherein the chirality of the starting material is preserved. An exemplary scheme is as follows:

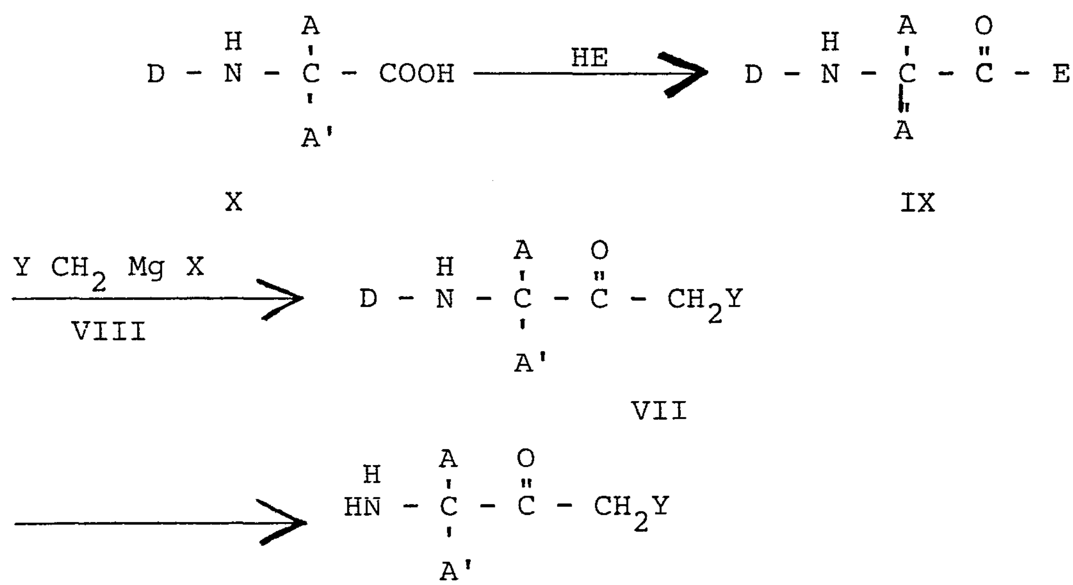


X

IX

VII

VI

wherein B' is an amino protecting group, B is a carboxy protecting group, A, A', Y and n have the same meaning as previously described, X is a halogen, such as Cl or Br, D is an amino protecting group, such as triphenylmethyl and E is a carboxylic acid protecting group, such as pyridyl thio ester. In this procedure, a protected amino acid of Formula IX, such as N-triphenyl methyl D-alanine pyridyl thio ester is reacted with a Grignard reagent of Formula VIII to produce the B-amino ketone of Formula VII. The protecting group D, i.e., the triphenyl methyl group, can be easily removed by techniques known in the art, such as by treating the compound of Formula VII with oxalic acid and acetic acid. The resulting deprotected product is then coupled with a protected α-aminodicarboxylic acid of Formula II in the usual fashion to form the product of Formula V.

The intermediate ketone of Formula V can also be prepared by condensing compounds of general Formula II (a protected α-aminodicarboxylic acid or acylating derivative thereof) with a salt of an amino ketone of Formula XII:

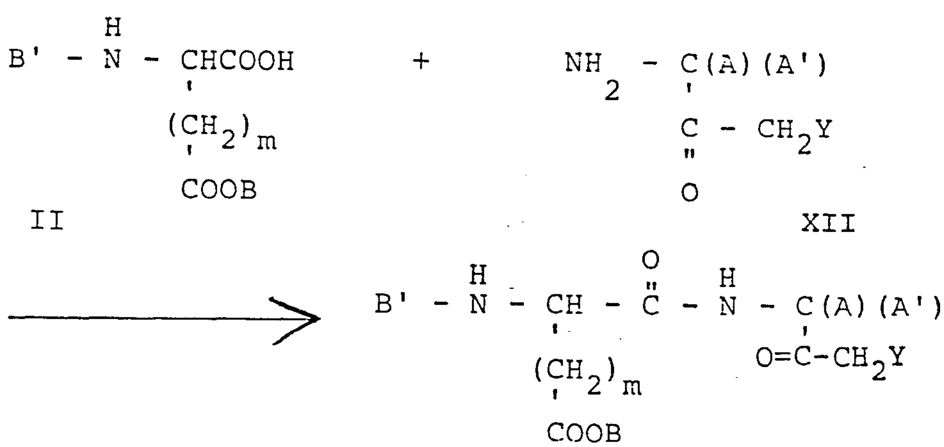

II

XII

Examplary salts of compounds of Formula XII include oxalates, malonates, succinates and the like.

In these, B' is an amino protecting group, B is a carboxy protecting group, and A, A', Y, and n have the same meaning as previously described. A variety of protecting groups known in the art may be employed. Examples of many of these possible groups may be found in "Protective Groups in Organic Synthesis" by T.W. Green, John Wiley and Sons, 1981. Among the preferred groups that may be employed are benzylcarbonxyl for A and benzyl for B.

Coupling of compounds with general formula II to compounds having general formula XII employs established techniques in peptide chemistry. One such technique uses dicyclohexylcarbodiimide (DCC) as the coupling agent. The DCC method may be employed with or without additives such as 4-dimethyl-aminopyridine or copper (II). The DCC coupling reaction generally proceeds at room temperature, however, it may be carried out from about -20° to 50°C. in a variety of solvents inert to the reactants. Thus suitable solvents include, but are not limited to, N,N-dimethyl-formamide, methylene chloride, toluene and the like. Preferably the reaction is carried out under an inert atmosphere such as argon or nitrogen. Coupling usually is complete within 2 hours but may take as long as 24 hours depending on reactants.

Various other amide-forming methods can be employed to prepare the desired compounds using suitable derivatives of the free-carboxy group in compounds of structure II, e.g., acid halide, mixed anhydride with acetic acid and similar derivatives. The following illustrates such methods using aspartic acid as the amino dicarboxylic acid.

One such method utilizes the reaction of N-protected aspartic anhydrides with the selected amino compound of formula XII. Thus compounds of formula XII can be reacted directly in inert organic solvents with L-aspartic anhydride having its amino group protected by a formyl, carbobenzloxy, or p-methoxycarbobenzloxy group which is subsequently removed after coupling to give compounds of general formula I. The N-acyl-L-aspartic anhydrides are prepared by reacting the corresponding acids with acetic anhydride in amounts of 1.0 - 1.2 moles per mole of the N-acyl-L-aspartic acid at 0° to 60°C in an inert solvent. The N-acyl-L-aspartic anhydrides are reacted with preferably 1 to 2 moles of compounds of formula XII in an organic solvent capable of dissolving both and inert to the same. Representative solvents are ethyl acetate, methyl propionate, tetrahydrofuran, dioxane, ethyl ether, N,N-dimethylformamide and benzene. The reaction proceeds smoothly at 0° to 30°C. The N-acyl group is removed after coupling by catalytic hydrogenation with palladium on carbon or with HBr or HCl in a conventional manner. U.S. Patent 3,879,372 discloses that this coupling method can also be performed in an aqueous solvent at a temperature of -10° to 50°C. and at a pH of 4-12.

Compounds of Formula XII can be prepared by techniques known in the art. In one reaction scheme, compounds of Formula XII are prepared by oxidation of alkenes of Formula XIII using conventional oxidizing agents, such as peroxy acids, e.g., meta-chloroperoxybenzoic acid:

$$NH_2 - \underset{\underset{C\,=\,CHY}{|}}{C}(A)(A') \xrightarrow{[O]} XII$$

XIII

in which A, A', and Y are as defined above. The alkene XIII in turn is prepared from the reaction of a protected α-amino aldehyde of Formula XIV and a Grignard reagent of Formula XV, followed by dehydration and removal of the protecting group.

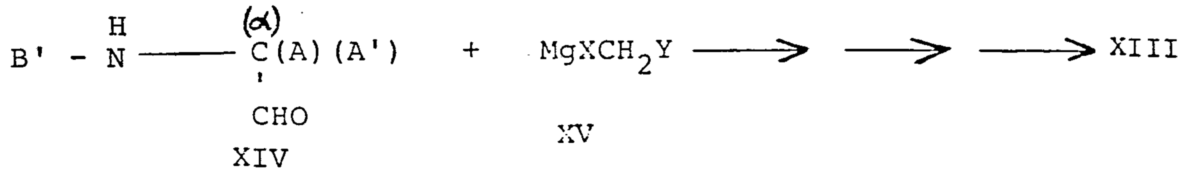

XIV XV

wherein B', (A), A' and Y are as defined hereinabove and X is a halogen, such as bromide or chloride.

Alternatively, compounds of Formula I can be prepared by selective reduction of the compounds of Formula XI by reduction techniques known in the art, such as by utilizing $NaBH_4$, $LiAlH_4$ or by catalytic hydrogenation. This reduction involves conversion of the $-\underset{O}{\underset{\|}{C}}-CH_2$ group to $-\underset{OH}{\underset{|}{CH}}-CH_2-$.

Compounds in which A = $CH_2OH$ are prepared by reduction of the carbo methoxy group in otherwise comparable products.

Methoxy methyl compounds are prepared by methylation (dimethylsulfate) of those in which A=$CH_2OH$.

Compounds of Formula XI can be prepared by the removel of the protecting groups from the carboxy moiety of Formula V and when necessary, from the amino moiety by techniques known in the art as discussed hereinbelow.

Alternatively, compounds of Formula XI can be prepared from the coupling of N - trifluoro acetyl - L - aspartic acid anhydride (XVII) with a salt of an amino ketone of Formula XII:

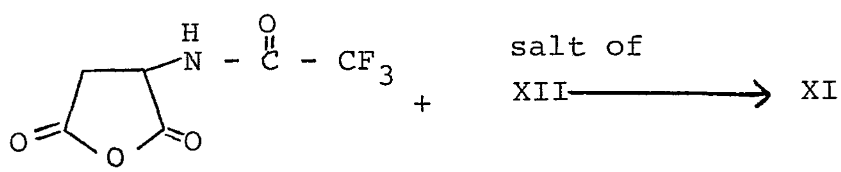

With regard to the removal of protecting groups from compounds of formulae IV and V a number of deprotecting techniques are known in the art and can be utilized to advantage depending on the nature of the protecting groups. Among such techniques is catalytic hydrogenation utilizing palladium on carbon or transfer hydrogenation with 1,4-cyclohexadiene. Generally the reaction is carried at room temperature but may be conducted from 5 to 65°C. Usually the reaction is carried out in the presence of a suitable solvent which may include, but are not limited to water, methanol, ethanol, dioxame, tetrahydrofuran, acetic acid, t-butyl alcohol, isopropanol or mixtures thereof. The reaction is usually run at a positive hydrogen pressure of 50 psi but can be conducted over the range of 20 to 250 psi. Reactions are generally quantitative taking 1 to 24 hours for completion.

In any of the previous synthetic methods the desired products are preferably recovered from reaction mixtures by crystallization. Alternatively, normal or reverse-phase chromatography may be utilized as well as liquid/liquid extraction or other means.

The desired compounds of formula I are usually obtained in the free acid form; they may also be recovered as their physiologically acceptable salts, i.e., the corresponding amino salts such as hydrochloride,sulfate hydrosulfate, nitrate, hydrobromide, hydroiodide, phosphate or hydrophosphate; or the alkali metal salts such as the sodium, potassium, lithium, or the alkaline earth metal salts such as calcium or magnesium, as well as aluminum, zine and like salts.

Conversion of the free peptide derivatives of formula I into their physiologically acceptable salts is carried out by conventional means, as for example, bringing the compounds of formula I into contact with a mineral acid, an alkali metal hydroxide, an alkali metal oxide or carbonate or an alkaline earth metal hydroxide, oxide, carbonate or other complexed form.

These physiologically acceptable salts can also be utilized as sweetness agents usually having increased solubility and stability over their free forms.

It is known to those skilled in the art that the compounds of the present invention having asymmetric carbon atoms may exist in racemic or optically active forms. All of these forms are contemplated within the scope of the invention.

The compounds of the present invention have two asymmetric sites, which are designated by an asterik (*) in the formula below, and one pseudo-asymmetric site which is designated by a double asterik (**):

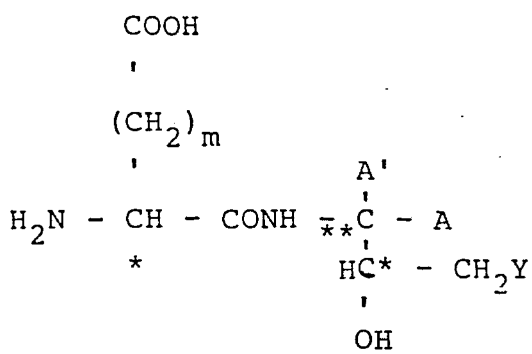

Whenever A is identical to A', the compounds of the present invention have only two asymmetric sites, designated by the asterisk in the dicarboxylic acid moiety and the secondary carbinol carbon. Although both the D and L forms are possible; the preferred compounds are those in which the dicarboxylic acid group is in the L-configuration. Whenever, the groups A' and A are different, the carbon atoms designated by the double asterisk become asymetric centers and the compounds of the present invention will contain at least three asymmetric centers. Further asymmetric centers can exist in the group ZY as is recognizable to those skilled in this art. Regardless, the configuration around each of the asymmetric sites, whenever present, may exist in either the D or L forms, and all possible stereoisomers are contemplated to be within the scope of the present invention.

The following examples further illustrate the invention.

EXAMPLE 1

N-(L-Aspartyl)-2-amino-2-methyl-4-(2,2,5,5-tetramethylcyclopentyl)-butan-3-ol

N-Boc-2-aminoisobutyric acid is dissolved in tetrahydrofuran and is stirred at 0°C. under argon. Bis-(N-methylpiperazinyl)-aluminum hydride is added and the reaction mixture is heated to reflux overnight. Ether is then added, and the excess hydride is quenched with saturated NaCl. The aqueous phase is separated and extracted with ether. The combined organic phases are washed with 2 M NaOH, 2 M HCl and saturated NaCl. The solution is dried over $Na_2SO_4$ and evaporated to yield N-Boc-2-amino-2-methylpropanal.

A solution of 2,2,5,5-tetramethyl-1-cyclopentylmethyl bromide in ether is added slowly to magnesium turnings until the Grignard reagent begins to form. The remainder of the alkyl bromide is then added and the mixture is stirred until all the magnesium dissolves. At 0°C. a solution of N-Boc-2-amino-2-methylpropanal is then added and the mixture is stirred overnight. The reaction is quenched with 1 M HCl, and extracted with ether and the extracts are evaporated to obtain N-Boc-2-amino-4-(2,2,5,5-tetramethyl-1-cyclopentyl)-butan-3-ol which is dissolved in trifluoroacetic acid and the solution is stirred overnight. Water is added and the mixture is made basic with 20% KOH. The mixture is extracted with ether and the organic layer is dried over $Na_2SO_4$ and evaporated to yield 2-amino-2-methyl-4-(2,2,5,5-tetramethyl-1-cyclopentyl)-butan-3-ol.

To a magnetically stirred solution of 2-amino-2-methyl-4-(2,2,5,5-tetramethyl-1-cyclopentyl)-butan-3-ol in dry dimethylformamide at 0°C. under argon atmosphere is added N-Cbz-L-aspartic acid beta-benzyl ester followed by copper (II) chloride and dicyclohexylcarbodiimide. This is stirred for 18 hours, after which the reaction mixture is poured into 0.1 N HCl and extracted with ethyl acetate. The organic phase is washed with saturated $NaHCO_3$ and then water, and is dried over $MgSO_4$. Evaporation of the solvent yields N-(N'-Cbz-L-aspartyl beta-benzyl ester)-2-amino-2-methyl-4-(2,2,5,5-tetramethyl-1-cyclopentyl)-butan-3-ol.

N-(N'-Cbz-L-Aspartyl beta-benzyl ester)-2-amino-2-methyl-4-(2,2,5,5-tetramethyl-1-cyclopentyl)-butan-3-ol is dissolved in absolute ethanol to give a 0.1 M solution. An equivalent weight of 10% palladium on carbon is added and the solution is cooled in an ultra-sound ice bath. Cyclohexadiene (10 equivalents) is added and the solution is sonicated. After the reaction is complete, as judged by thin layer chromatography, the mixture is filtered through Celite with ethanol and evaporated to yield the deprotected product.

Similarly, using the appropriate starting materials, the following additional compounds are prepared:

N-L-aspartyl-2-amino-2-methyl-4-(2,2,4,5-tetramethylcyclopentyl)butan-3-ol;
N-L-aspartyl-2-amino-2-methyl-4-(2,2,5-trimethylcyclopentyl)butan-3-ol;
N-L-aspartyl-2-amino-2-methyl-4-(2,5-dimethylcyclopentyl)butan-3-ol;
N-L-aspartyl-2-amino-2-methyl-4-(dicyclopropylmethyl)-butan-3-ol;
N-L-aspartyl-2-amino-2-methyl-4-(fenchyl)butan-3-ol;
N-L-aspartyl-2-amino-2-methyl-4-(2-t-butylcyclopentyl)-butan-3-ol;
N-L-aspartyl-2-amino-2-methyl-4-(di-t-butylmethyl)butan-3-ol;
N-L-aspartyl-2-amino-2-methyl-4-(diisopropylmethyl)-butan-3-ol;
N-(L-aspartyl)-2-amino-4-(diisopropylmethyl)butan-3-ol;
N-(L-aspartyl)-2-amino-4-(di-t-butylmethyl)butan-3-ol.

EXAMPLE 2

Preparation of alpha-L-aspartyl-2-amino-2-methyl-3-hydroxy-alkanes

Preparation of N-boc-2-amino-2-methyl-1-propanol

To a stirred solution 6g (0.067 mol) of the amino-alcohol in 250 mls of saturated of $NaHCO_3$ was added 16.15g (0.074 mol) of di-tert-butylcarbonate in 75 mls of tert-butanol at room temperature. The reaction was allowed to stir overnight. TLC (4:1/hexane-ethyl acetate) showed a ninhydrin positive compound at $R_f$=0.5. The reaction was extracted twice with ethyl acetate, dried over anhydrous $MgSO_4$, and concentrated in vacuo. Purification with flash chromatography yielded 6 g (50.6%) of the desired product.

Preparation of N-boc-2-amino-methyl-1-propanal

To a solution of blocked amino-alcohol (3g, 0.016 mol) in triethylamine (6.62 mls, 0.0476 mol) at room temperature was added with stirring sulfur trioxide-pyridium complex (7.6 g, 0.0476 mol) in 20 mls of methyl-sulfoxide (DMSO). After five minutes, the oxidation was complete as indicated by TLC (4:1/hexane-ethyl acetate). The brown solution was diluted with 100 mls of ethyl acetate and washed three times with 75 ml portions of water. The organic layer was then washed with two 50 ml portion of 10% citric acid, one 50 ml portion of saturated $NaHCO_3$, and finally two 75 ml portions of water. The organic layer was then dried over $MgSO_4$ and concentrated to afford 1.88g (63%) of the aldehyde as a white, wax-like solid. NMR ($CDCl_3$): $\delta$ 9.4 (s, 1H, CHO), 4.6 - 5.0 (broad s, 1H, NH, 1.4 (s, 9H, tert-butyl), 1.3 (s, 6H, $2CH_3$).

Preparation of N-boc-2-amino-2-methyl-3-hydroxy-methylcyclohexane

Pre-treated magnesium turnings (0.027g, 0.009 mol) were placed in a three necked flash under argon. Anhydrous diethyl ether was introduced to the flask as to just submerse the magnesium. Dropwise addition of cyclohexylmethyl bromide to initiate the reaction followed. The mixture was stirred for 0.5 h with gentle heating and 50 mls of ether was added. Several minutes passed and dropwise addition of the remainder of the bromide (1.26 ml, 0.0053 mol) followed. Stirring under gentle reflux continued for 2 h, and the reaction mixture was then cooled to room temperature. Double needle transfer of the Grignard to a 1M solution of the aldehyde (1g, 0.0053 mol) at 0°C followed by a gradual warming to room temperature and finally to a gentle reflux resulted in a reaction mixture with many components. TLC (6:1/hexane-ethyl acetate) showed much of the aldehyde remained unchanged, however, persistent refluxing resulted in more side reactions. The reaction was quenched with 50 mls of cold 1M HCl and diluted with 100 mls of ether. The organic layer was separated and washed with 50 mls of saturated $NaHCO_3$, 50 mls of water, and dried over $MgSO_4$. Concentration and purification by flash chromatography afforded 0.12g (8.3%) of the secondary alcohol. NMR ($CDCl_3$):$\delta$4.55 - 4.75 (broad s, 1H, NH), 3.4 - 4.0 (dt, 1H, CCHOH), 1.4 (s, 9H, tert-butyl), 1.3 (s, 3H, $CH_3$), 1.15 (s, 3H, $CH_3$), 1.0 - 2.0 (broad m, 14H, OH, $CH_2$, ring).

Preparation of 2-amino-2-methyl-3-hydroxy-methylcyclohexane

A stirring solution of 0.12g (0.00042 mol) of the blocked amino-alcohol, 2.3 mls of absolute ethanol, 1.5 mls of water, and 0.085 mls of concentrated HCl was allowed to react for 2 h at reflux. TLC (6:1/hexane-ethyl acetate) showed a small impurity at the solvent front and a ninhydrin

positive compound at the origin. The mixture was allowed to cool to room temperature and was then diluted with 1M HCl. The solution was then made basic with NaOH pellets and extracted twice with ethyl acetate. The organic extractions were dried over $MgSO_4$ powder, filtered, and concentrated to afford 0.07g (90.9%) of a yellow oil.

Preparation of N-cbz-alpha-L-aspartic-beta benzyl ester-2-amino-2-methyl-3-hydroxy-methylcyclohexane

The amino-alcohol (0.07g, 0.00037 mol) was dissolved in 50 mls of THF and was reacted with 0.025 g (0.0005 mol) of N-cbz-L-aspartic-alpha-(para-nitrophenylester)-beta-benzylester for 24 h. TLC (2:1/hexane-ethyl acetate) showed a more polar compound than the aspartyl derivative at Rf=0.5. The solvent was removed under vacuum and the yellow oil was purified by flash chromatography to afford 0.1g (38.2%) of a glazy, white solid. NMR ($CDCl_3$):$\delta$ 7.25 - 7.4 (s, 10H, aromatic), 6.4 - 6.5 (broad s, 1H, $C(=O)NHC(CH_3)_2$), 5.9 - 6.1 (d, 1H, NHcbz), 5.1 (s, 4H, $CH_2$ benzyls), 4.3 - 4.7 (broad m, 1H, $CH_2CHNHcbz$), 3.4 - 3.8 (broad m, 1H, CCHOH), 2.7 - 3.0 (dd, 2H, $C(=O)CH_2CH$), 0.6 - 2.0 (broad m, 20H, $2CH_3$, OH, $CH_2$, ring).

Preparation of alpha-L-aspartyl-2-amino-2-methyl-3-hydroxy-methylcyclohexane

The bis-blocked compound (0.1g, 0.00019 mol) was dissolved in 100 mls of methanol and placed in a hydrogenation vessel. The vessel was purged with argon and a catalytic amount of 10% Pd-C was added. Hydrogen gas was passed through the system for 3 minutes at 55 PSI when TLC (2:1/hexane-ethyl acetate) revealed no starting material remained. The mixture was filtered through Celite, concentrated, and purified by reversed phase flash chromatography employing 70% methanol-water as the eluant. FAB MS (m/z): (M+) 301 (72%), 187 (17%), 168 (70%), 133 (60%), 111 (38%), 88 (100%).

EXAMPLE 3

Using the procedure of Examples 1 and 2 and utilizing the appropriate starting materials. Alpha -L-aspartyl-2-amino-2-methyl-3-hydroxy-4-isobutylbutane was prepared. Physical data for the bis-blocked compound shows: NMR ($CDCl_3$): $\delta$ 7.3 (s, 10H, aromatic), 6.4 (broad s, 1H, C(=O)NHCH), 5.65 - 5.9 (d, 1H, NHcbz), 5.1 (s, 4H, $CH_2$ benzyl), 4.3 - 4.7 (broad m, 1H, $CH_2$CHNHcbz), 3.4 - 3.8 (broad m, 1H, CCHOH), 2.7 - 3.0 (m, SH, C(=O)$CH_2$CH)), 0.7 - 1.6 (broad m, 18H, $2CH_3$, OH, isobutane).

The bis blocked compound was converted to the desired product by removing the blocking groups as described in Example 1.

## EXAMPLE 4

Generalized Procedure for the chiral products

The starting material, N-triphenylmethyl D-alanine 2-pyridyl thioester, was prepared by treating a suspension of N-triphenylmethyl D-alanine (1 equiv.) $[\alpha]_D^{25}$ + 19.0° and 2-mercaptopyridine (1 equiv.) in ethyl acetate at 0°C with a solution of dicyclohexylcarbodiimide (1 equiv.) in ethyl acetate. The mixture was stirred overnight at room temperature and the dicyclohexylurea was removed by filtration. The filtrate was washed with saturated sodium hydrogen carbonate, water and brine. The organic layer was dried with sodium sulfate and rotary evaporated to give an oil which crystallized upon chromatography on silica gel with 3:1 petroleum ether/ ethyl acetate. The product, N-triphenylmethyl D-alanine 2-pyridyl thioester, was characterized by NMR spectroscopy and FAB mass spectroetry.

NMR ($CDCl_3$): $\delta$ 1.05(d,3H), 2.40 (d,1H), 3.65(m.1H), 7.15-7.70(m,18H), 8.55(m,1H); $[\alpha]_D^{25}$=98.5° (lit:$[\alpha]_D^{25}$=100.1°

Alkyl Halide

The requisite alkyl bromide, in the specific example of the patchanyl substituent, was prepared by a three step procedure from commercially available dl-patchenol (Aldrich Chem. Co.). Patchenol (50g) was dissolved in acetone (250ml) under argon in a Paar hydrogenation bottle. 10% Palladium on carbon (3g) was added and hydrogenation carried out at 50 psi. When hydrogen gas uptake was complete the mixture was filtered through Celite to give a 1.2 M solution of patchanol.

dl-patchanol

NMR ($CDCl_3$): $\delta$ 0.8-1.0(m, 6H), 1.10-1.75(m,9H), 1.85-2.15(br. s, 1H), 3.45-3.70 (m,2H).

The patchanol solution was cooled to 0°C in an ice-salt bath and sufficient Jones reagent (8 M) was added dropwise slowly to the vigorously stirred vessel When the orange color of the reagent persisted, the solution was taken up in cold water (1 L) and diethyl ether (500 mL). The green color was removed by an additional wash with water. The organic layer was extracted with 2N NaOH (1 L) and washed once again with diethyl ether. The aqueous phase was acidified to pH 2 with 6N HCl and extracted with diethyl ether (1 L). The organic layer was dried with sodium sulfate and evaporated to give 34.6 g of patchanoic acid.

Hunsdiecker Reaction

Patchanoic acid (3.64 g) and red mercuric oxide (2.4 g) were heated at 60°C in carbon tetrachloride (100 mL) while a solution of bromine (1 mL) in carbon tetrachloride (20 mL) was added. After refluxing for one hour the pink suspension was filtered through Celite and the filtrate was washed with 0.02 N NaOH (100 mL). The organic layer was dried with sodium sulfate and rotary evaporated to give a liquid which was distilled at 70°C and 1.5 mm Hg (76% yield). The product, exo/endo-2-bromomethyl 3,3-dimethyl norbornane, was characterized by NMR spectroscopy and gas chromatography/mass spectrometry. NMR ($CDCl_3$)I $\delta$ 0.95(dd,6H), 1.0-1.90(m,8H), 2.25(dd,1H), 3.20-3.50(m,2H).

Grignard Reaction to form the chiral ketone

To a dry flask under argon equipped with a reflux condenser and addition funnel was added freshly etched and dry magnesium turnings (2.6 equiv.) and dry tetrahydrofuran. A crystal of iodine was added and the solution was heated to 60°C while a tetrahydrofuran solution of the alkyl bromide (2.5 equiv.) was added dropwise over 45 minutes. When the magnesium had been consumed (ca. 2-3 h) the greyish solution was transferred via double syringe under argon to a flask at 0°C containing the N-triphenylmethyl D-alanine 2-pyridyl thioester in tetrahydrofuran. After one hour of stirring the reaction mixture was quenched with saturated aqueous ammonium chloride and extracted with ethyl acetate. The organic layer was dried with sodium sulfate and evaporated. The residue was chromatographed on silica gel with 30:1 petroleium ether/ ethyl acetate to give the desired chiral N-protected amino ketone. Using this method, and the appropriate starting material, D-2-N-triphenyl methyl amino-4-cyclohexyl 3-butanone was prepared in 85% yield. $[\alpha]_D^{25}$=80.8°, EtOAc; m/z 412(13.6), 334(32.7), 243(100), 165(30.9), 115(18.2).

Deprotection

The N-triphenylmethyl group was removed by mixing the ketone (1 equiv.) with oxalic acid (1.05 equiv.) in a solution of 1 part water to 2 parts glacial acetic acid overnight at room temperature. The mixture was filtered to remove the insoluble triphenylmethanol and the filtrate was hi-vacuum rotary evaporated to give a white solid. The product, as the ammonium oxalate salt, was characterized by NMR spectroscopy and FAB mass spectrometry.

D-2-amino 4-(cyclohexyl) 3-butanone

m/z 170(100), 55(25.7). (spectra is the free amine as dissolved in a glycerol matrix).

Coupling

The amino ketone was coupled to N-Cbz L-aspartic acid beta-benzyl ester by the classical mixed anhydride method employing N-methylmorpholine and iso-butyl chloroformate. The usual workup afforded an oil which crystallized upon chromatography on silica gel with 2:1 petroleum ether/ethyl acetate. The product, N-Cbz L-aspartic acid beta-benzyl ester alpha-2-amino (4-substituted) 3-butanone was characterized by NMR spectroscopy and FAB mass spectrometry.

Reduction of the Amido ketones to Amido alcohols

The amido ketone from above was dissolved in 95% ethanol at 0°C. Cerium trichloride (hydrate) 4 equiv. was added followed by the slow addition of 4 equiv. of sodium, borohydride. The milky solution was stirred for one hour at 0°C and judged to be completed by TLC. The contents of the flask were poured into sufficient water and ethyl acetate to break the emulsion. The organic layer was dried and rotary evaporated to give a white solid. The pure product was characterized by NMR spectroscopy and FAB mass spectrometry.

Deprotection

The products from above were dissolved in methanol under argon and 10% palladium on carbon was added. Hydrogenation at 50 psi was carried out in a Paar apparatus and when hydrogen gas uptake was complete the contents of the vessel were filtered through Celite with methanol. Evaporation was done at 2mm Hg and 30°C to give a white powder. The taste samples were

obtained by purification on $C_{18}$ reversed phase silica with combinations of methanol and water.

In those cases where the D-enriched alanines were prepared it was found that severe insolubility in methanol of the $C_2$ L-diastereomers continually precipitated them out of solution. Thus, isolations of the pure products were enriched in the more soluble $C_2$ D-diastereomers.

NMR Assignments and FAB/MS fragmentation ($M+H^+$) data:

1. N-(alpha-L-aspartyl)(DL)-2-amino 3-hydroxy 5,5-dimethyl hexane

($CD_3OD$): $\delta$ 0.95(s,9H), 1.05(d,3H), 1.65-1.75(dd,2H), 2.20 (br.s,1H), 2.50-2.70(q. of d,2H), 3.40-3.60(m,1H), 3.80-3.95 (m,1H), 3.95-4.05(q. of d,1H).

m/z 261 (54.3), 146(31.4), 128(100), 111(50.4), 88(43.8), 70(43.8), 55(51.4).

2. N-(alpha-L-aspartyl)(DL)-2-amino 3-hydroxy 6-methyl heptane

($CD_3OD$): $\delta$ 0.85((d,6H), 1.05(d,3H), 1.15-1.65(m,5H), 2.20 (br.s,1H), 2.50-2.70(q. of d,2H), 3.40-3.60(m,1H), 3.80-3.95 (m,1H), 3.95-4.05(q. of d,1H)

m/z 261(100), 146(43.7), 128(98.4), 111(16.4), 98(20.3), 88(57.8), 70(78.1), 55(63.3).

3. N-(alpha-L-aspartyl) DL-2-amino 3-hydroxy 4-cyclopentyl butane

($CD_3OD$): $\delta$ 1.15(dd,3H), 1.45-1.75(m,11H), 2.20(br.s,1H), 2.50-2.70(q. of d,2H), 3.40-3.60(m,1H), 3.80-3.95(m,1H), 3.95-4.05(q. of d,1H).

m/z 273(100), 255(14.8), 158(21.8), 140(58.6), 123(11.7), 88(38.3), 70(21.1), 55(29.7).

4. N-(alpha-L-aspartyl) DL-2-amino 3-hydroxy 4-cyclohexyl butane

($CD_3OD$): $\delta$ 1.15(dd,3H), 1.45-1.75(m,13H), 2.20(br.s,1H), 2.50-2.70(q. of d,2H), 3.40-3.60(m,1H), 3.80-3.95(m,1H), 3.95-4.05(q. of d.1H).

m/z 287(100), 269(17.9), 172(35.1), 154(64.8), 95(44.5), 88(44.5), 70(42.9), 55(99.2).

BAD ORIGINAL

5. N-(alpha-L-aspartyl) D-2-amino 3-hydroxy 4-cyclohexyl butane

($CD_3OD$): $\delta$ 1.15(dd,3H), 1.45-1.75(m,13H), 2.20(br.s,1H), 2.50-2.70(q. of d,2H), 3.40-3.60(m,1H), 3.80-3.95(m,1H), 3.95-4.05(q. of d,1H).

m/z 287(100), 172(32.0), 154(90.6), 133(17.9), 115(14.0), 88(62.5), 70(45.3), 55(100).

6. N-(alpha-L-aspartyl) L-2-amino 3-hydroxy 4-cyclohexyl butane

($CD_3OD$): $\delta$ 1.15(dd,3H), 1.45-1.75(m,13H), 1.45-1.55(dd,2H), 2.20(br.s,1H), 2.50-2.70(q. of d,2H), 3.40-3.60(m,1H), 3.80-3.95(m,1H), 3.95-4.05(q. of d,1H).

m/z 287(40.6), 243(19.5), 172(23.4), 154(62.5), 115(12.5), 95(45.3), 81(52.3), 70(40.6), 55(100).

7. N-(alpha-L-aspartyl) DL-2-amino 3-hydroxy 4-adamantyl butane

($CD_3OD$): $\delta$ 1.15(dd,3H), 1.40-1.80(m,15H), 1.65-1.75(dd,2H), 2.20(br.s,1H), 2.50-2.70(q. of d,2H), 3.40-3.60(m,1H), 3.80-3.95(m,1H), 3.95-4.05(q. of d,1H).

m/z 339(28.1), 206(15.6), 185(28.9), 135(23.4), 93(100). 75(43.7), 57(37.5).

8. N-(alpha-L-aspartyl) DL-2-amino 3-hydroxy 4-[(exo/endo)-2-norbornyl] butane

($CD_3OD$): $\delta$ 1.15(dd,3H), 1.40-1.80(m,11H), 1.65-1.75(dd,2H), 2.20(br.s,1H), 3.95-4.05(q. of d.1H).

m/z 299(100), 281(10.1), 184(14.0), 166(44.5), 149(21.8), 95(37.5), 88(24.2), 67(46.8), 55(31.2).

9. N-(alpha-L-aspartyl) DL-2-amino 3-hydroxy 4-[(exo/endo)-2-norbornyl] butane

($CD_3OD$): $\delta$ 1.15(dd,3H), 1.40-1.80(m,11H), 1.65-1.75(dd,2H), 2.20(br.s,1H), 2.50-2.70(q. of d,2H), 3.40-3.60(m,1H), 3.80-3.95(m.1H), 3.95-4.05(q. of d,1H).

m/z 299(64.3), 281(8.6), 184(17.8), 166(68.9), 149(20.9), 91(100), 67(75.9), 55(55.0).

BAD ORIGINAL

10. N-(alpha-L-aspartyl) DL-2-amino 3-hydroxy 4-[2-(3,3-dimethyl) exo/endo-norbornyl] butane

($CD_3OD$): δ 0.080-1.05(m. of s,6H), 1.05-1.15(dd,3H), 1.15-1.40 (m,6H), 1.40-1.65(m,3H), 1.65-1.75(dd,2H), 2.20(br.s,1H), 2.50-2.70(q. of d,2H), 3.40-3.60(m,1H), 3.80-3.95(m,1H), 3.95-4.05(q. of d,1H).

m/z 327(100), 212(14.0), 194(42.1), 177(15.6), 133(20.3), 88(44.5), 70(34.3), 55(42.9).

11. N-(alpha-L-aspartyl) DL-2-amino 3-hydroxy 4-[2-(6,6-dimethyl) exo/endo-bicyclo(3.1.1)heptyl] butane

($CD_3OD$): δ 1.00-(s,3H), 1.15(s,3H), 1.20-1.25(dd,3H), 1.15-1.40(br.m,9H) 1.65-1.75(dd,2H), 2.20(br.s,1H), 2.50-2.70(q. of d,2H), 3.40-3.60(m,1H), 3.80-3.95(m,1H), 3.95-4.05(q. of d, 1H).

m/z 327(67.2), 194(20.3), 177(16.4), 133(19.5), 107(21.1), 81(72.6), 67(100), 55(96.8).

12. N-(alpha-L-aspartyl) DL-2-amino 3-hydroxy 4-ethyloxy butane

($CD_3OD$): δ 1.15(d,3H), 1.20(t,3H), 2.20(br.s,1H), 2.75(d,2H), 2.95(d,2H), 3.5(q,2H), 3.40-3.60(m,1H), 3.80-3.95(m,1H), 3.95-4.05(q. of d,1H).

m/z 249(100), 155(14.8), 134(50.7), 116(14.8), 99(15.6), 88(32.8), 70(52.3), 55(27.3).

13. N-(alpha-L-aspartyl) DL-2-amino 3-hydroxy 6-methyl (trans)-4-heptene

($CD_3OD$): δ 0.95(d,6H), 1.05(d,3H), 1.65(m,1H), 2.05-2.30(m,1H), 2.20(br.s,1H), 2.50-2.70(q. of d,2H), 3.80-3.95(m,1H), 3.95-4.05(q. of d,1H), 5.30-5.60(dd,2H).

m/z 281(29.7), 259(22.6), 241(39.0), 126(100), 109(58.6), 91(88.3), 70(46.8), 55(46.8).

BAD ORIGINAL

Sweetness determinations with the present compounds gave the following results:

1. Aspartyl-2-amino-2-methyl-3-hydroxy-4-cyclohexylbutane

| % Compound | Sucrose Eq. | X-Sucrose |
|---|---|---|
| .025 | 1.7 | 67 |

2. Aspartyl-2-amino-3-hydroxy-4-t-butylbutane

| % Compound | Sucrose Eq. | X-Sucrose |
|---|---|---|
| .05 | 1.75 | 35 |

3. Aspartyl-2-amino-3-hydroxy-4-isobutylbutane

| % Compound | Sucrose Eq. | X-Sucrose |
|---|---|---|
| .1 | 5.5 | 55 |

4. Aspartyl-2-amino-3-hydroxy-4-cyclopentylbutane

| % Compound | Sucrose Eq. | X-Sucrose |
|---|---|---|
| .05 | 6.5 | 130 |
| .1 | 10 | 100 |

5. Aspartyl-2-amino-3-hydroxy-4-isobut-1-enylbutane

| % Compound | Sucrose Eq. | X-Sucrose |
|---|---|---|
| .05 | 2.17 | 43 |
| .1 | 3.17 | 32 |

6. Aspartyl-2-amino-2-methyl-3-hydroxy-4-isobutylbutane

| % Compound | Sucrose Eq. | X-Sucrose |
|---|---|---|
| .025 | 1.75 | 70 |
| .05 | 3.0 | 60 |
| .1 | 4.5 | 45 |

7. Aspartyl-2-amino-3-hydroxy-4-norbornylbutane

| % Compound | Sucrose Eq. | X-Sucrose |
|---|---|---|
| .005 | 1.00 | 200 |
| .01 | 2.75 | 275 |
| .025 | 5.5 | 220 |

8. Aspartyl-2-amino-3-hydroxy-4-adamantylbutane

| % Compound | Sucrose Eq. | X-Sucrose |
|---|---|---|
| .025 | 1.0 | 40 |
| .05 | 2.0 | 40 |
| .1 | 3.0 | 30 |

9. Aspartyl-D-2-amino-3-hydroxy-4-cyclohexylbutane

| % Compound | Sucrose Eq. | X-Sucrose |
|---|---|---|
| .01 | 2.25 | 225 |
| .025 | 5.25 | 210 |
| .05 | 8.00 | 160 |

10. Aspartyl-2-amino-3-hydroxy-4-nopanylbutane

| % Compound | Sucrose Eq. | X-Sucrose |
|---|---|---|
| .005 | 1.7 | 333 |
| .01 | 2.7 | 267 |
| .025 | 5.0 | 200 |
| .05 | 6.8 | 137 |

11. Aspartyl-2-amino-3-hydroxy-4-patchanylbutane

| % Compound | Sucrose Eq. | X-Sucrose |
|---|---|---|
| .005 | 3.17 | 634 |
| .01 | 5.00 (7.67) | 500 (767) |
| .025 | 9.00 | 360 |

(the higher numbers on .01% are for a retasting of an extensively dried sample)

EXAMPLE 5

N-(L-Aspartyl)-3-amino-1-(2,2,5,5-tetramethyl-1-cyclopentyl)-2-butanone

Methoxymethyltriphenylphosphonium chloride is suspended in tetrahydrofuran at 0°C. under argon. Sec-Butyllithium in cyclohexane is added, followed by a solution of 2,2,5,5-tetramethylcyclopentanone in tetrahydrofuran. After one hr. water is added to the reaction mixture. The organic layer is separated, washed with water, dried over $MgSO_4$ and evaporated to yield the enol ether. The ether is dissolved in dioxane and 2 M $H_2SO_4$ is added. The mixture is refluxed until the reaction is complete, as judged by thin layer chromatography. The mixture is poured into water and extracted with ether. The organic layer is dried over $MgSO_4$ and evaporated to yield 2,2,5,5-tetramethylcyclopentane-1-carboxaldehyde.

2,2,5,5-Tetramethylcyclopentane-1-carboxaldehyde is dissolved in 95% ethanol and sodium borohydride is added. After 24 hours, the reaction is quenched with 1 M HCl and extracted with ether. The extract is washed, dried over $MgSO_4$ and evaporated to yield 2,2,5,5-tetramethyl-1-cyclopentylmethanol.

2,2,5,5-Tetramethyl-1-cyclopentylmethanol is dissolved in benzene and stirred at 0°C. under argon. A solution of phosphorus tribromide in benzene is added and the mixture is stirred for 2 hours and then heated to 60°C. for 4 hours. The mixture is cooled, poured into ice and extracted with ether. The organic layer is washed with saturated $NaHCO_3$, dried over $MgSO_4$ and evaporated to yield 2,2,5,5-tetramethyl-1-cyclopentylmethyl bromide.

N-Boc-D-Alanine is dissolved in tetrahydrofuran and stirred at 0°C. under argon. Bis-(N-methylpiperazinyl)-aluminum hydride is then added, and the excess hydride quenched with saturated NaCl. The aqueous phase is separated and extracted with ether. The combined organic phases are washed with 2 M NaOH, 2 M HCl and saturated NaCl. The solution is dried over $NaSO_4$ and evaporated to yield N-Boc-D-alaninal.

A solution of 2,2,5,5-tetramethyl-1-cyclopentylmethyl bromide in ether is added slowly to magnesium turnings until the Grignard reagent begins to form. The remainder of the alkyl bromide is then added and the mixture is stirred until all the magnesium has dissolved. At 0°C. a solution of N-Boc-D-alaninal is then added and the mixture is stirred overnight. The reaction is quenched with 1 M HCl, extracted with ether and the extracts evaporated. The residue is dissolved in dioxane and 2 M $H_2SO_4$ is added. The mixture is heated until the alcohol is dehydrated as shown by thin layer chromatography. Water is added and the mixture extracted with ether. The organic layer is dried over $MgSO_4$ and evaporated to give N-Boc-2-amino-4-(2,2,5,5-tetramethyl-1-cyclopentyl)-3-butene.

N-Boc-2-amino-4-(2,2,5,5-tetramethyl-1-cyclopentyl)-3-butene is dissolved in ether to which is added an ether solution of meta-chloroperbenzoic acid. After the oxidation is complete as judged by thin layer chromatography the reaction is diluted with ether and washed twice with dilute $NaHCO_3$. After drying over $MgSO_4$ the solvent is evaporated to give the crude epoxide. The epoxide is redissolved in dry ether in

BAD ORIGINAL

a flask equipped with a reflux condenser and an argon gas inlet. To this solution is added an equivalent amount of anhydrous magnesium bromide and the contents of the flask, refluxed for 4 hours under argon. The mixture is then cooled, poured over ice and extracted with ethyl acetate. The organic layer is dried over $Na_2SO_4$ and filtered. The filtrate is evaporated to give two major products which are separated by column chromatography on silica gel to yield N-Boc-2-amino-4-(2,2,5,5-tetramethyl-1-cyclopentyl)-3-butanone and N-Boc-2-amino-4-oxo-4-(2,2,5,5-tetramethyl-1-cyclopentyl)butone.

N-Boc-2-amino-4-(2,2,5,5-tetramethyl-1-cyclopentyl)-3-butanone is dissolved in trifluoroacetic acid and the solution is stirred overnight. Water is added and the mixture is made basic with 20% KOH. The mixture is extracted with ether, the organic layer dried over $Na_2SO_4$ and evaporated to yield 2-amino-4-(2,2,5,5-tetramethyl-1-cyclopentyl)-3-butanone.

To a magnetically stirred solution of 2-amino-4-(2,2,5,5-tetramethyl-1-cyclopentyl)-3-butanone in dry dimethylformamide at 0°C. under argon atmosphere is added N-Cbz-L-aspartic acid beta-benzyl ester followed by copper (II) chloride and dicyclohexylcarbodiimide. This is stirred for 18 hours, after which the reaction mixture is poured into 0.1 N HCl and extracted with ethyl acetate. The organic phase is washed with saturated $NaHCO_3$ and then water, and dried over $MgSO_4$. Evaporation of the solvent yields N-(N'-Cbz-L-aspartyl-beta-benzyl ester)-2-amino-4-(2,2,5,5-tetramethyl-1-cyclopentyl)-3-butanone.

N-(N'-Cbz-L-Aspartyl beta-benzyl ester)-2-amino-4-(2,2,5,5-tetramethyl-1-cyclopentyl)-3-butanone is dissolved in absolute ethanol to give a 0.1 M solution. An equivalent weight of 10% palladium on carbon is added and the solution cooled in an ultra-sound ice bath. Cyclohexadiene (10 equivalents) is added and sonification begins. After the reaction is complete, as judged by thin layer chromatography, the mixture is filtered through Celite with ethanol and evaporated to yield N-(L-aspartyl)-2-amino-4-(2,2,5,5-tetramethyl-1-cyclopentyl)-3-butanone.

EXAMPLE 6

N-(L-aspartyl)-3-amino-3-methyl-1-(2,2,5,5-tetramethyl-1-cyclopentyl)-2-butanone

Methoxymethyltriphenylphosphonium chloride is suspended in tetrahydrofuran at 0°C. under argon. *sec*-Butyllithium in cyclohexane is added, followed by a solution of 2,2,5,5-tetramethylcyclopentanone in tetrahydrofuran. After one hour, water is added to the reaction mixture. The organic layer is separated, washed with water, dried over $MgSO_4$ and evaporated to yield the enol ether. The ether is dissolved in dioxane and 2M $H_2SO_4$ is added. The mixture is refluxed until the reaction is complete, as judged by thin layer chromatography. The mixture is poured into water and extracted with ether. The organic layer is dried over $MgSO_4$ and evaporated to yield 2,2,5,5-tetramethylcyclopentane-1-carboxaldehyde.

2,2,5,5-Tetramethylcyclopentane-1-carboxaldehyde is dissolved in 95% ethanol and sodium borohydride is added. After 24 hours, the reaction is quenched with 1 M HCl and extracted with ether. The extract is washed, dried over $MgSO_4$ and evaporated to yield 2,2,5,5-tetramethyl-1-cyclopentylmethanol.

2,2,5,5-Tetramethyl-1-cyclopentylmethanol is dissolved in benzene and stirred at 0°C. under argon. A solution of phosphorus tribromide in benzene is added and the mixture stirred for 2 hours, and then heated to 60°C. for 4 hours. The mixture

is cooled, poured into ice and extracted with ether. The organic layer is washed with saturated $NaHCO_3$, dried over $MgSO_4$ and evaporated to yield 2,2,5,5-tetramethyl-1-cyclopentylmethyl bromide.

N-Boc-2-aminoisobutyric acid is dissolved in tetrahydrofuran and stirred at 0°C. under argon. Bis-(N-methylpiperazinyl)-aluminum hydride is added and the reaction mixture heated to reflux overnight. Ether is then added, and the excess hydride quenched with saturated NaCl. The aqueous phase is separated and extracted with ether. The combined organic phases are washed with 2 M NaOH, 2 M HCl and saturated NaCl. The solution is dried over $Na_2SO_4$ and evaporated to yield N-Boc-2-amino-2-methylpropanol.

A solution of 2,2,5,5-tetramethyl-1-cyclopentylmethyl bromide in ether is added slowly to magnesium turnings until the Grignard reagent begins to form. The remainder of the alkyl bromide is then added and the mixture stirred until all the magnesium dissolves. At 0°C. a solution of N-Boc-2-amino-2-methylpropanol is then added and the mixture is stirred overnight. The reaction is quenched with 1 M HCl, extracted with ether and the extracts evaporated. The residue is dissolved in dioxane and 2 M $H_2SO_4$ is added. The mixture is heated until the alcohol is dehydrated as shown by thin layer chromatography. Water is added and the mixture extracted with ether. The organic layer is dried over $MgSO_4$ and evaporated to give N-Boc-2-amino-2-methyl-4-(2,2,5,5-tetramethyl-1-cyclopentyl)butene.

N-Boc-2-amino-2-methyl-4-(2,2,5,5-tetramethyl-1-cyclopentyl)butene is dissolved in ether to which is added an ether solution of meta-chloroperbenzoic acid. After the oxidation is complete, as judged by thin layer chromatography, the reaction is diluted with ether and washed twice with dilute $NaHCO_3$. After drying over $MgSO_4$ the solvent is evaporated to give the crude epoxide. The epoxide is redissolved in dry ether in a flask equipped with a reflux condenser and an argon gas inlet. To this solution is added an equivalent amount of anhydrous magnesium bromide and the contents of the flask refluxed for 4 hours under argon. The mixture is then cooled, poured over ice and extracted with ethyl acetate. The organic layer is dried over $NaSO_4$ and filtered. The filtrate is evaporated to give two major products which are separated by column chromatography on silica gel to yield N-Boc-2-amino-2-methyl-4-(2,2,5,5-tetramethyl-1-cyclopentyl)-3-butanone and -Boc-2-amino-4-oxo-4-(2,2,5,5-tetramethyl-1-cyclopentyl) butane.

N-Boc-2-amino-2-methyl-4-(2,2,5,5-tetramethyl-1-cyclopentyl)-3-butanone is dissolved in trifluoroacetic acid and the solution is stirred overnight. Water is added and the mixture is made basic with 20% KOH. The mixture is extracted with ether and the organic layer is dried over $Na_2SO_4$ and evaporated to yield 2-amino-2-methyl-4-(2,2,5,5-tetramethyl-1-cyclopentyl)-3-butanone.

To a magnetically stirred solution of 2-amino-2-methyl-4-(2,2,5,5-tetramethyl-1-cyclopentyl)-3-butanone in dry dimethylformamide at 0°C. under argon atmosphere is added N-Cbz-L-aspartic acid beta-benzyl ester followed by copper (II) chloride and dicyclohexylcarbodiimide. This is stirred for 18 hours, after which the reaction mixture is poured into 0.1 N HCl and extracted with ethyl acetate. The organic phase is washed with saturated $NaHCO_3$ and then water, and dried over $MgSO_4$. Evaporation of the solvent yields N-(N'-Cbz-L-Aspartyl beta-benzyl ester)-2-amino-2-methyl-4-(2,2,5,5-tetramethyl-1-cyclopentyl)-3-butanone.

N-(N'-Cbz-L-Aspartyl beta-benzyl ester)-2-amino-2-methyl-4-(2,2,5,5-tetramethyl-1-cyclopentyl)-3-butanone is dissolved in absolute ethanol to give 0.1 M solution. An equivalent weight of 10% palladium on carbon is added and the solution cooled in an ultra sound ice bath. Cyclohexadiene (10 equivalents) is added and sonication begun. After the reaction is complete as judged by thin layer chromatography, the mixture is filtered through Celite with ethanol and evaporated to yield N-(L-aspartyl)-2-amino-2-methyl-4-(2,2,5,5-tetramethyl-1-cyclopentyl)-3-butanone.

In a similar manner using appropriate starting materials, the following ketones were also prepared:

aspartyl-2-amino-4 patchanyl-3-butanone
aspartyl-2-amino-4-nopanyl-3-butanone
aspartyl-D-2-amino-4-cyclohexyl-3-butanone
aspartyl-2-amino-4-norbornyl-3-butanone

Example 7

N-2-(N-trifluoroacetyl)-2-aspartyl-D-2-amino-4-cyclohexyl-3 butanone

A. N-Trifluoroacetyl L-aspartyl acid anhydride

Prepared according to literature methods (F. Weygand, Chem. Ber. 1957, 90, 1986. and Proctor and Gamble Eur. Pat. Appl. (1980) 0013044). In our hands, we obtained a 90% yield, mp 135°C $[\alpha]^{25}_{D}$ -20.2° in TMF $[\alpha]^{25}_{D}$ -22.3 in TMF (literature).

B. N-α-(N-Trifluoro acetyl) L-aspartyl D-2-amino 4-cyclohexyl 3-butanone

The amine oxalate salt of D-2-amino-4-cyclohexyl-3-butanone was dissolved in dry tetrahydrofuran (2mL) and N-trifluoroacetyl-L-aspartic acid anhydride (1/equiv.) was added. Stirred at room temperature overnight. Evaporation at reduced pressure afforded a solid which was crystallized twice from a minimum amount of water. Reversed phase HPLC with 50% methanol/water slowed the α:B ration equal to 4:1. The desired product was characterized by FAB mass spectrometry M/Z (m+m 381, 204, 140, 112, Sweetness = 80 x sucrose.

CLAIMS

1. A compound represented by the formula:

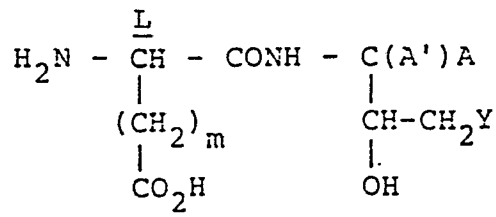

wherein

A is hydrogen, alkyl containing 1-3 carbon atoms, hydroxyalkyl containing 1-3 carbon atoms, alkoxymethyl wherein the alkoxy contains 1-3 carbon atoms or $CO_2R_5$ in which $R_5$ is alkyl containing 1-3 carbon atoms;

A' is hydrogen or alkyl containing 1-3 carbon atoms;

A and A' taken together with the carbon atoms to which they are attached form cycloalkyl containing 3-4 carbon atoms;

Y is $-(CHR)_n-R_1$, $-CHR_2R_3$ or $R_4$;

$R_1$ is cycloalkyl, cycloalkenyl, lower alkyl substituted cycloalkyl or cycloalkenyl, bicycloalkyl, bicycloalkenyl or tricycloalkyl containing up to 10 ring carbon atoms and up to a total of 12 carbon atoms;

R is H or alkyl containing 1-4 carbon atoms;

$R_2$ and $R_3$ are each cycloalkyl containing 3-4 ring carbon atoms;

$R_4$ is alkyl containing up to 12 carbon atoms;

n = 0 or 1; and

m = 0 or 1;

and food-acceptable salts thereof.

2. A compound represented by the formula:

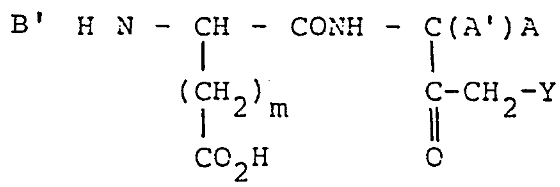

wherein

A is hydrogen, alkyl containing 1-3 carbon atoms, hydroxyalkyl containing 1-3 carbon atoms, alkoxymethyl wherein the alkoxy contains 1-3 carbon atoms or $CO_2CH_3$;

A' is hydrogen or alkyl containing 1-3 carbon atoms;

A and A' taken together with the carbon atom to which they are attached form cycloalkyl containing 3-4 carbon atoms;

Y is $-(CHR)_n-R_1$, $-CHR_2R_3$ or $R_4$;

$R_1$ is cycloalkyl, cycloalkenyl, lower alkyl substituted cycloalkyl or cycloalkenyl, bicycloalkyl, bicycloalkenyl or tricycloalkyl containing up to 10 ring carbon atoms and up to a total of 12 carbon atoms;

R is H or alkyl containing 1-4 carbon atoms;

$R_2$ and $R_3$ are each cycloalkyl containing 3-4 ring carbon atoms;

$R_4$ is alkyl containing up to 12 carbon atoms;

B′ is hydrogen or an amine protecting group

n = 0 or 1; and

and food-acceptable salts thereof.

3. A compound according to Claim 1 or 2 wherein $R_1$ is cyclopentyl or cyclohexyl containing a total of up to 10 carbon atoms.

4. A compound according to Claims 1, 2 or 3 wherein n = 0.

5. A compound according to Claims 1, 2 or 4 wherein $R_1$ is mono-, di-, tri- or tetramethyl cycloalkyl or bicycloalkyl containing up to 10 carbon atoms.

6. A compound according to Claims 1, 2, 4 or 5 wherein $R_1$ is a $\beta$-methyl-substituted cycloalkyl or bicycloalkyl.

7. A compound according to Claims 1, 2, 4 or 5 wherein $R_1$ is $\beta$, $\beta$ or $\beta$, $\beta'$-dimethyl-substituted cycloalkyl or bicycloalkyl.

8. A compound according to Claims 1, 2, 4 or 5 wherein $R_1$ is a $\beta$, $\beta$, $\beta'$-trimethyl-substituted cycloalkyl or bicycloalkyl.

9. A compound according to Claims 1, 2, 4 or 5 wherein $R_1$ is a $\beta$, $\beta$, $\beta'$, $\beta'$-tetramethyl-substituted cycloalkyl or bicycloalkyl.

10. A compound according to Claims 1-9 wherein $R_2$ and $R_3$ are cyclopropyl.

11. A compound according to Claims 1-10 wherein $R_4$ is di-t-butylmethyl, diisopropylmethyl or di-t-amylmethyl.

12. A compound according to Claims 1-11 wherein A and A' are each H or $CH_3$.

13. A compound according to Claims 1-11 wherein A is alkoxymethyl containing 1-3 carbon atoms in the alkoxy group.

14. A compound according to Claims 1-11 wherein A is carbomethoxy.

15. A compound according to Claims 1-14 wherein m = 1.

16. A compound according to Claims 1 or 2 wherein $R_4$ is $-CH(R_6)(R_7)$ wherein $R_6$ and $R_7$ are each alkyl.

17. A compound according to Claim 16 wherein $R_6$ and $R_7$ are branched-chain alkyls.

18. The compound according to Claims 1 or 2 wherein Y is 2-methylcyclopentyl and A is $-CO_2CH_3$;

Y is 2,5-dimethylcyclopentyl and A is $CO_2CH_3$;

Y is 2,2,5-trimethylcyclopentyl and A is $CO_2CH_3$;

Y is 2,2,5,5-tetramethylcyclopentyl and A is $CO_2CH_3$;

Y is 2-t-butylcyclopentyl and A is $CO_2CH_3$;

Y is di-t-butylmethyl and A is $CO_2CH_3$;

Y is diisopropylmethyl and A is $CO_2CH_3$;

Y is 1-cyclopropyl-1-t-butylmethyl and A is $CO_2CH_3$;

Y is 2-methylcyclopentyl and A and A' are each H or $CH_3$;

Y is 2,5-dimethylcyclopentyl and A and A' are each H or $CH_3$;

Y is 2,2,5-trimethylcyclopentyl and A and A' are each H or $CH_3$;

Y is 2,2,5,5-tetramethylcyclopentyl and A and A' are each H or $CH_3$;

Y is 2-t-butylcyclopentyl and A and A' are each H or $CH_3$;

Y is di-t-butylmethyl and A and A' are each H or $CH_3$;

Y is diisopropylmethyl and A and A' are each H or $CH_3$;

Y is 1-cyclopropyl-1-t-butylmethyl and A and A' are each H or $CH_3$;

Y is 2-methylcyclopentyl and A and A' together with the carbon atom to which they are attached form a cyclopropyl group;

Y is 2,5-dimethylcyclopentyl and A and A' together with the carbon atoms to which they are attached form a cyclopropyl group;

Y is 2,2,5-trimethylcyclopentyl and A and A' together with the carbon atoms to which they are attached form a cyclopropyl group;

Y is 2,2,5,5-tetramethylcyclopentyl and A and A' together with the carbon atoms to which they are attached form a cyclopropyl group;

Y is 2-t-butylcyclopentyl and A and A' together with the carbon atom to which they are attached form a cyclopropyl group;

Y is di-t-butylmethyl and A and A' together with the carbon atom to which they are attached form a cyclopropyl group;

Y is diisopropylmethyl and A and A' together with the carbon atom to which they are attached form a cyclopropyl group;

Y is 1-cyclopropyl-1-t-butylmethyl and A and A' together with the carbon atom to which they are attached form a cyclopropyl group;

Y is 2-methylcyclopentyl and A is methoxyalkyl;

Y is 2,5-dimethylcyclopentyl and A is methoxyalkyl;

Y is 2,2,5-trimethylcyclopentyl and A is methoxyalkyl;

Y is 2,2,5,5-tetramethylcyclopentyl and A is methoxyalkyl;

Y is 2-t-butylmethyl and A is methoxyalkyl;

Y is di-t-butylmethyl and A is methoxyalkyl;

Y is diisopropylmethyl and A is methoxyalkyl; or

Y is 1-cyclopropyl-1-t-butylmethyl and A is methoxyalkyl

19. The compound according to Claims 1 or 2 wherein Y is norbornyl, cyclohexyl, nopanyl, or patchanyl.

20. The compound according to Claim 1 which is aspartyl-2-amino-3-hydroxy-4-patchanylbutane; aspartyl-2-amino-3-hydroxy-4-nopanylbutane; aspartyl-D-2-amino-3-hydroxy-cyclonexyl-butane; or aspartyl-2-amino-3-hydroxy-4-norbornylbutane.

21. The compound according to Claim 2 which is aspartyl-2-amino-4-patchanyl-3-butanone; aspartyl-2-amino-4-nopanyl-3-butanone; aspartyl-D-2-amino-4-cyclohexyl-3-butanone; or aspartyl-2-amino-4-norbornyl-3-butanone.

22. The compound according to Claims 1 or 2 having an N-amino protecting group or a carboxy protecting group.

23. The compound according to Claim 22 wherein A is benzyloxycarbonyl and B is benzyl.

24. An edible composition comprising a sweetening effective amount of a compound according to Claim 1.

Claims for Austria

1. A method for the preparation of a sweetening compound, characterized in that a compound represented by the formula:

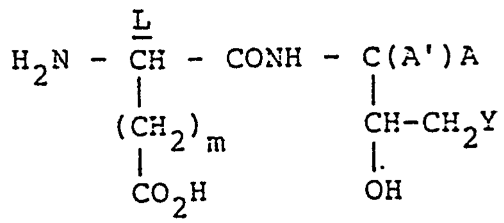

wherein

A is hydrogen, alkyl containing 1-3 carbon atoms, hydroxyalkyl containing 1-3 carbon atoms, alkoxymethyl wherein the alkoxy contains 1-3 carbon atoms or $CO_2R_5$ in which $R_5$ is alkyl containing 1-3 carbon atoms;

A' is hydrogen or alkyl containing 1-3 carbon atoms;

A and A' taken together with the carbon atoms to which they are attached form cycloalkyl containing 3-4 carbon atoms;

Y is $-(CHR)_n-R_1$, $-CHR_2R_3$ or $R_4$;

$R_1$ is cycloalkyl, cycloalkenyl, lower alkyl substituted cycloalkyl or cycloalkenyl, bicycloalkyl, bicycloalkenyl or tricycloalkyl containing up to 10 ring carbon atoms and up to a total of 12 carbon atoms;

R is H or alkyl containing 1-4 carbon atoms;

$R_2$ and $R_3$ are each cycloalkyl containing 3-4 ring carbon atoms;

$R_4$ is alkyl containing up to 12 carbon atoms;

n = 0 or 1; and

m = 0 or 1;

and food-acceptable salts thereof,

is prepared by amide-forming methods known per se.

2. A method for the preparation of a sweetening compound, characterized in that a compound represented by the formula:

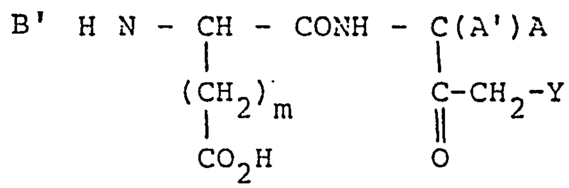

wherein

A is hydrogen, alkyl containing 1-3 carbon atoms, hydroxyalkyl containing 1-3 carbon atoms, alkoxymethyl wherein the alkoxy contains 1-3 carbon atoms or $CO_2CH_3$;

A' is hydrogen or alkyl containing 1-3 carbon atoms;

A and A' taken together with the carbon atom to which they are attached form cycloalkyl containing 3-4 carbon atoms;

Y is $-(CHR)_n-R_1$, $-CHR_2R_3$ or $R_4$;

$R_1$ is cycloalkyl, cycloalkenyl, lower alkyl substituted cycloalkyl or cycloalkenyl, bicycloalkyl, bicycloalkenyl or tricycloalkyl containing up to 10 ring carbon atoms and up to a total of 12 carbon atoms;

R is H or alkyl containing 1-4 carbon atoms;

$R_2$ and $R_3$ are each cycloalkyl containing 3-4 ring carbon atoms;

$R_4$ is alkyl containing up to 12 carbon atoms;

B' is hydrogen or an amine protecting group

n = 0 or 1; and

and food-acceptable salts thereof,

is prepared by amide-forming methods known per se.

3. The method according to claim 1 or 2, wherein $R_1$ is cyclopentyl or cyclohexyl containing a total of up to 10 carbon atoms.

0 203 540

4. The method according to claims 1, 2 or 3, wherein n = 0.

5. The method according to claims 1, 2 or 4, wherein $R_1$ is mono-, di-, tri- or tetramethyl cycloalkyl or bicycloalkyl containing up to 10 carbon atoms.

6. The method according to claims 1, 2, 4 or 5, wherein $R_1$ is a ß-methyl-substituted cycloalkyl or bicycloalkyl.

7. The method according to claims 1, 2, 4 or 5, wherein $R_1$ is a ß,ß or ß,ß'-dimethyl-substituted cycloalkyl or bicycloalkyl.

8. The method according to claims 1, 2, 4 or 5, wherein $R_1$ is a ß,ß,ß'-trimethyl-substituted cycloalkyl or bicycloalkyl.

9. The method according to claims 1, 2, 4 or 5, wherein $R_1$ is a ß,ß,ß',ß'-tetramethyl-substituted cycloalkyl or bicycloalkyl.

10. The method according to claims 1 to 9, wherein $R_2$ and $R_3$ are cyclopropyl.

11. The method according to claims 1 to 10, wherein $R_4$ is di-t-butylmethyl, diisopropylmethyl or di-t-amylmethyl.

12. The method according to claims 1 to 11, wherein A and A' are each H or $CH_3$.

13. The method according to claims 1 to 11, wherein A is alkoxymethyl containing 1 to 3 carbon atoms in the alkoxy group.

14. The method according to claims 1 to 11, wherein A is carbomethoxy.

15. The method according to claims 1 to 14, wherein m = 1.

16. The method according to claims 1 or 2, wherein $R_4$ is $-CH(R_6)(R_7)$ wherein $R_6$ and $R_7$ are each alkyl.

17. The method according to claim 16, wherein $R_6$ and $R_7$ are branched-chain alkyls.

18. The method according to claims 1 or 2, wherein Y is 2-methylcyclopentyl and A is $-CO_2CH_3$;

Y is 2,5-dimethylcyclopentyl and A is $CO_2CH_3$;

Y is 2,2,5-trimethylcyclopentyl and A is $CO_2CH_3$;

Y is 2,2,5,5-tetramethylcyclopentyl and A is $CO_2CH_3$;

Y is 2-*t*-butylcyclopentyl and A is $CO_2CH_3$;

Y is di-*t*-butylmethyl and A is $CO_2CH_3$;

Y is diisopropylmethyl and A is $CO_2CH_3$;

Y is 1-cyclopropyl-1-*t*-butylmethyl and A is $CO_2CH_3$;

Y is 2-methylcyclopentyl and A and A' are each H or $CH_3$;

Y is 2,5-dimethylcyclopentyl and A and A' are each H or $CH_3$;

Y is 2,2,5-trimethylcyclopentyl and A and A' are each H or $CH_3$;

Y is 2,2,5,5-tetramethylcyclopentyl and A and A' are each H or $CH_3$;

Y is 2-*t*-butylcyclopentyl and A and A' are each H or $CH_3$;

Y is di-*t*-butylmethyl and A and A' are each H or $CH_3$;

Y is diisopropylmethyl and A and A' are each H or $CH_3$;

Y is 1-cyclopropyl-1-*t*-butylmethyl and A and A' are each H or $CH_3$;

Y is 2-methylcyclopentyl and A and A' together with the carbon atom to which they are attached form a cyclopropyl group;

Y is 2,5-dimethylcyclopentyl and A and A' together with the carbon atoms to which they are attached form a cyclopropyl group;

Y is 2,2,5-trimethylcyclopentyl and A and A' together with the carbon atoms to which they are attached form a cyclopropyl group;

Y is 2,2,5,5-tetramethylcyclopentyl and A and A' together with the carbon atoms to which they are attached form a cyclopropyl group;

Y is 2-t-butylcyclopentyl and A and A' together with the carbon atom to which they are attached form a cyclopropyl group;

Y is di-t-butylmethyl and A and A' together with the carbon atom to which they are attached form a cyclopropyl group;

Y is diisopropylmethyl and A and A' together with the carbon atom to which they are attached form a cyclopropyl group;

Y is 1-cyclopropyl-1-t-butylmethyl and A and A' together with the carbon atom to which they are attached form a cyclopropyl group;

Y is 2-methylcyclopentyl and A is methoxyalkyl;

Y is 2,5-dimethylcyclopentyl and A is methoxyalkyl;

Y is 2,2,5-trimethylcyclopentyl and A is methoxyalkyl;

Y is 2,2,5,5-tetramethylcyclopentyl and A is methoxyalkyl;

Y is 2-t-butylmethyl and A is methoxyalkyl;

Y is di-t-butylmethyl and A is methoxyalkyl;

Y is diisopropylmethyl and A is methoxyalkyl; or

Y is 1-cyclopropyl-1-t-butylmethyl and A is methoxyalkyl

19. The method according to claims 1 or 2, wherein Y is norbornyl, cyclohexyl, nopanyl, or patchanyl.

20. The method according to claim 1, wherein the sweetening compound is
aspartyl-2-amino-3-hydroxy-4-patchanylbutane;
aspartyl-2-amino-3-hydroxy-4-nopanylbutane;
aspartyl-D-2-amino-3-hydroxy-cyclonexyl-butane; or
aspartyl-2-amino-3-hydroxy-4-norbornylbutane.

21. The method according to claim 2, wherein the sweetening compound is
aspartyl-2-amino-4-patchanyl-3-butanone;
aspartyl-2-amino-4-nopanyl-3-butanone;
aspartyl-D-2-amino-4-cyclohexyl-3-butanone; or
aspartyl-2-amino-4-norbornyl-3-butanone.

22. The method according to claims 1 or 2, wherein the sweetening compound is provided with an N-amino protecting group or a carboxy protecting group.

23. The method according to claim 22, wherein A is benzyloxycarbonyl and B is benzyl.

24. A method for the preparation of an edible composition characterized in that a sweetening effective amount of a compound obtained by the method of claim 1, is admixed to the remaining composition.

European Patent Office

**EUROPEAN SEARCH REPORT**

0 203 540

EP 86 10 6992

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int Cl 4) |
| Y | EP-A-0 013 044 (THE PROCTER & GAMBLE)<br>* Claims * | ~~1,24~~ | C 07 C 103/50<br>C 07 C 125/065<br>A 23 L 1/236 |
| Y | JOURNAL OF MEDICINAL CHEMISTRY, vol. 24, no. 5, May 1981, pages 572-583, American Chemical Society, US; H. IWAMURA: "Structure-sweetness relationship of L-aspartyl dipeptide analogues. A receptor site topology"<br>* Whole document * | 1,24 | |
| D,Y | DE-A-1 906 048 (SEARLE)<br>* Claims * | 1,24 | |
| D,Y | EP-A-0 068 551 (THE PROCTER & GAMBLE)<br>* Claims * | 1,24 | TECHNICAL FIELDS SEARCHED (Int Cl 4)<br>C 07 C 103/00<br>A 23 L 1/00<br>C 07 C 125/00 |

The present search report has been drawn up for all claims

| Place of search | Date of completion of the search | Examiner |
|---|---|---|
| THE HAGUE | 29-08-1986 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82